# EUROPEAN PATENT APPLICATION

(11) **EP 4 134 431 A1**
(43) Date of publication of application: **15.02.2023**
(21) Application number: 21785350.6
(22) Date of filing: 05.04.2021
(51) Int. Cl.: C12N 5/10, C12N 1/15, C12N 1/19, C12N 1/21, C12N 15/09

(54) **GENOME ALTERATION METHOD AND GENOME ALTERATION KIT**

(30) Priority: 06.04.2020 JP 2020068266; 25.08.2020 JP 2020141335
(71) Applicant: Logomix, Inc., Tokyo 104-0053 (JP); Tokyo Institute of Technology, Tokyo 152-8550 (JP)
(72) Inventor: AIZAWA, Yasunori, Tokyo 152-8550 (JP); OHNO, Tomoyuki, Tokyo 152-8550 (JP)
(74) Representative: Hoffmann Eitle
(86) International application number: PCT/JP2021/014495
(87) International publication number: WO 2021/206054

(57) **Abstract**

Provided are a genome engineering method and a genome engineering kit which can efficiently engineer two or more alleles and are capable of engineering a relatively large region. The present invention provides a genome engineering method for engineering two or more alleles, comprising the steps of: (a) introducing the following (i) and (ii) to a cell comprising the chromosome: (i) a genome engineering system comprising a sequence-specific nucleic acid cleaving molecule targeting a target region in the chromosomal genome, or a polynucleotide encoding the sequence-specific nucleic acid cleaving molecule, and (ii) two or more donor DNAs for selective markers respectively having different selective marker genes (the number of types of the donor DNAs for selective markers are equal to or more than the number of the alleles that are subject to genome engineering); and (b) selecting the cell on the basis of all the selective marker genes carried by the two or more donor DNAs for selective markers.

## Description

### [Technical Field]

The present invention relates to a genome engineering method and a genome engineering kit.

### [Background Art]

Since a CRISPR/Cas system was reported as a novel genome editing tool, various studies have been conducted using the CRISPR/Cas system (e.g., Patent Literature 1). In the genome editing using the CRISPR/Cas system, a target region targeted by guide RNA undergoes double-strand break by Cas9 nuclease. It is known that the DNA that thus has undergone double-strand break is repaired by homology directed repair (HDR) or non-homologous end-joining repair (NHEJ). In HDR, an arbitrary sequence can be integrated into the target region by introducing a donor DNA having a sequence homologous to a neighboring region of the target region, together with the CRISPR/Cas system, to cells.

### [Citation List]

### [Patent Literature]

[Patent Literature 1] International Publication No. WO 2014/093661

### [Summary of Invention]

### [Technical Problem]

Conventional genome engineering techniques fail to efficiently engineer two or more alleles at the same time by HDR. Furthermore, these techniques have a limitation in the size of a genomic region that can be engineered by HDR, and find difficulty in efficiently performing the large-scale engineering of the genome (e.g., 10 kbp).

Accordingly, an object of the present invention is to provide a genome engineering method and a genome engineering kit which can efficiently engineer two or more alleles and are capable of engineering a relatively large region.

### [Solution to Problem]

As one example, the present invention includes the following aspects.
[1] A genome engineering method for engineering two or more alleles in a chromosomal genome, comprising the steps of:
   (a) introducing the following (i) and (ii) to a cell comprising the chromosome:
      (i) a genome engineering system comprising a sequence-specific nucleic acid cleaving molecule targeting a target region in the chromosomal genome, or a polynucleotide encoding the sequence-specific nucleic acid cleaving molecule, and
      (ii) two or more donor DNAs for selective markers, each of which comprises a nucleotide sequence of a selective marker gene between an upstream homology arm having a nucleotide sequence homologous to an upstream nucleotide sequence adjacent to the target region and a downstream homology arm having a nucleotide sequence homologous to a downstream nucleotide sequence adjacent to the target region, the two or more donor DNAs for selective markers respectively having different selective marker genes, wherein the number of types of the donor DNAs for selective markers is equal to or more than the number of the alleles that are subject to genome engineering; and
   (b) after the step (a), selecting the cell on the basis of all the selective marker genes carried by the two or more donor DNAs for selective markers.
[2] The genome engineering method according to [1], wherein the selective marker gene is a positive selective marker gene, and the step (b) is the step of selecting a cell expressing the same number of the positive selective marker gene as the number of the alleles.
[3] The genome engineering method according to [2], wherein each of the donor DNAs for selective markers further has a negative selective marker gene between the upstream homology arm and the downstream homology arm.
[4] The genome engineering method according to [3], further comprising the steps of: (c) after the step (b), introducing, to the cell, a donor DNA for recombination comprising a desired nucleotide sequence between an upstream homology arm having a nucleotide sequence homologous to an upstream nucleotide sequence adjacent to the target region and a downstream homology arm having a nucleotide sequence homologous to a downstream nucleotide sequence adjacent to the target region; and (d) after the step (c), selecting a cell not expressing the negative selective marker gene.
[5] The genome engineering method according to [3] or [4], wherein the positive selective marker gene is a drug resistance gene, and the negative selective marker gene is a fluorescent protein gene.
[6] The genome engineering method according to any one of [1] to [5], wherein the sequence-specific nucleic acid cleaving molecule is sequence-specific endonuclease.
[7] The genome engineering method according to [6], wherein the genome engineering system comprises Cas protein, and guide RNA having a nucleotide sequence homologous to a nucleotide sequence within the target region.
[8] A genome engineering kit for engineering two or more alleles in the chromosomal genome, comprising the following (i) and (ii):
   (i) a genome engineering system comprising a sequence-specific nucleic acid cleaving molecule targeting a target region in the chromosomal genome, or a polynucleotide encoding the sequence-specific nucleic acid cleaving molecule, and
   (ii) two or more donor DNAs for selective markers, each of which comprises a nucleotide sequence of a selective marker gene between an upstream homology arm having a nucleotide sequence homologous to an upstream nucleotide sequence adjacent to the target region and a downstream homology arm having a nucleotide sequence homologous to a downstream nucleotide sequence adjacent to the target region, the two or more donor DNAs for selective markers respectively having different selective marker genes, wherein the number of types of the donor DNAs for selective markers is equal to or more than the number of the alleles that are subject to genome engineering.
[9] The genome engineering kit according to [8], wherein the selective marker gene is a positive selective marker gene.
[10] The genome engineering kit according to [9], wherein each of the donor DNAs for selective markers further has a negative selective marker gene between the upstream homology arm and the downstream homology arm.
[11] The genome engineering kit according to any one of [8] to [10], wherein the sequence-specific nucleic acid cleaving molecule is sequence-specific endonuclease.
[12] The genome engineering kit according to any one of [8] to [11], wherein the genome engineering system comprises Cas protein, and guide RNA having nucleotide sequence homologous to a nucleotide sequence within the target region.

As one example, the present invention includes the following aspects.
[1] A method for preparing a cell in which two or more alleles in the chromosomal genome are engineered, comprising the steps of:
   (a) introducing the following (i) and (ii) to a cell comprising two or more alleles to introduce a selective marker gene to each of the two or more alleles:
      (i) a genome engineering system comprising a sequence-specific nucleic acid cleaving molecule capable of targeting a target region in the two or more alleles in the chromosomal genome and cleaving the target region, or a polynucleotide encoding the sequence-specific nucleic acid cleaving molecule, and
      (ii) two or more donor DNAs for selective markers, each of which has an upstream homology arm having a nucleotide sequence that can be homologously recombined with an upstream nucleotide sequence of the target region and a downstream homology arm having a nucleotide sequence that can be homologously recombined with a downstream nucleotide sequence of the target region, and comprises a nucleotide sequence of the selective marker gene between the upstream homology arm and the downstream homology arm, the two or more donor DNAs for selective markers respectively having distinguishably different selective marker genes, wherein the selective marker gene is unique to each type of donor DNA for the selective marker, and the number of types of the donor DNAs for selective markers is equal to or more than the number of the alleles that are subject to genome engineering; and (b) after the step (a), selecting a cell having the distinguishably different unique selective marker genes, which are respectively introduced in the two or more alleles by respectively homologously recombining the two or more alleles with different types of donor DNAs for selective markers, the cell expressing all the distinguishably different selective marker genes thus introduced (step for positive selection).
[2] A method for engineering two or more alleles in a chromosomal genome, comprising the steps of:
   (a) introducing the following (i) and (ii) to a cell comprising two or more alleles to introduce a selective marker gene to each of the two or more alleles:
      (i) a genome engineering system comprising a sequence-specific nucleic acid cleaving molecule capable of targeting a target region in the two or more alleles in the chromosomal genome and cleaving the target region, or a polynucleotide encoding the sequence-specific nucleic acid cleaving molecule, and
      (ii) two or more donor DNAs for selective markers, each of which has an upstream homology arm having a nucleotide sequence that can be homologously recombined with an upstream nucleotide sequence of the target region and a downstream homology arm having a nucleotide sequence that can be homologously recombined with a downstream nucleotide sequence of the target region, and comprises a nucleotide sequence of the selective marker gene between the upstream homology arm and the downstream homology arm, the two or more donor DNAs for selective markers respectively having distinguishably different selective marker genes, wherein the selective marker gene is unique to each type of donor DNA for the selective marker, and the number of types of the donor DNAs for selective markers is equal to or more than the number of the alleles that are subject to genome engineering; and (b) after the step (a), selecting a cell having the distinguishably different unique selective marker genes, which are respectively introduced in the two or more alleles by respectively homologously recombining the two or more alleles with different types of donor DNAs for selective markers, the cell expressing all the distinguishably different selective marker genes thus introduced (step for positive selection).
[3] The method according to [1] or [2], wherein the target region has a length of 5 kbp or more.
[4] The method according to [3], wherein the target region has a length of 8 kbp or more.
[5] The method according to any of [1] to [4], wherein
   each of the two or more donor DNAs for selective markers has a selective marker gene for positive selection, a marker gene for negative selection, and a target sequence between the upstream homology arm and the downstream homology arm, wherein in the case of using the selective marker gene both for positive selection and for negative selection, another selective marker gene for negative selection is optionally absent,
   the method further comprising the steps of:
      (c) after the step (b), introducing the following (iii) and (iv) to the selected cell to introduce a donor DNA for recombination to each of the two or more alleles:
         (iii) a genome engineering system comprising a sequence-specific nucleic acid cleaving molecule capable of targeting the target sequence and cleaving the target sequence, or a polynucleotide encoding the sequence-specific nucleic acid cleaving molecule, and
         (iv) a donor DNA for recombination comprising a desired nucleotide sequence, the donor DNA for recombination having an upstream homology arm having a nucleotide sequence that can be homologously recombined with an upstream nucleotide sequence of the target region and a downstream homology arm having a nucleotide sequence that can be homologously recombined with a downstream nucleotide sequence of the target region; and
      (d) after the step (c), selecting a cell not expressing the marker gene for negative selection (step for negative selection).
[6] The method according to [3], wherein
   each of the two or more donor DNAs for selective markers has a selective marker gene for positive selection, a marker gene for negative selection, and a target sequence between the upstream homology arm and the downstream homology arm, wherein in the case of using the selective marker gene both for positive selection and for negative selection, another selective marker gene for negative selection is optionally absent,
   the method further comprising the steps of:
      (c) after the step (b), introducing the following (iii) and (iv) to the selected cell to introduce a donor DNA for recombination to each of the two or more alleles:
         (iii) a genome engineering system comprising a sequence-specific nucleic acid cleaving molecule capable of targeting the target sequence and cleaving the target sequence, or a polynucleotide encoding the sequence-specific nucleic acid cleaving molecule, and
         (iv) a donor DNA for recombination comprising a desired nucleotide sequence, the donor DNA for recombination having an upstream homology arm having a nucleotide sequence that can be homologously recombined with an upstream nucleotide sequence of the target region and a downstream homology arm having a nucleotide sequence that can be homologously recombined with a downstream nucleotide sequence of the target region; and
      (d) after the step (c), selecting a cell not expressing the marker gene for negative selection (step for negative selection).
[7] The method according to [4], wherein
   each of the two or more donor DNAs for selective markers has a selective marker gene for positive selection, a marker gene for negative selection, and a target sequence between the upstream homology arm and the downstream homology arm, wherein in the case of using the selective marker gene both for positive selection and for negative selection, another selective marker gene for negative selection is optionally absent,
   the method further comprising the steps of:
      (c) after the step (b), introducing the following (iii) and (iv) to the selected cell to introduce a donor DNA for recombination to each of the two or more alleles:
         (iii) a genome engineering system comprising a sequence-specific nucleic acid cleaving molecule capable of targeting the target sequence and cleaving the target sequence, or a polynucleotide encoding the sequence-specific nucleic acid cleaving molecule, and
         (iv) a donor DNA for recombination comprising a desired nucleotide sequence, the donor DNA for recombination having an upstream homology arm having a nucleotide sequence that can be homologously recombined with an upstream nucleotide sequence of the target region and a downstream homology arm having a nucleotide sequence that can be homologously recombined with a downstream nucleotide sequence of the target region; and
      (d) after the step (c), selecting a cell not expressing the marker gene for negative selection (step for negative selection).
[8] The method according to any of [5] to [7], wherein the region between the upstream homology arm and the downstream homology arm of the donor DNA for recombination has a length of 5 kbp or more.
[9] The method according to [6] or [7], wherein the region between the upstream homology arm and the downstream homology arm of the donor DNA for recombination has a length of 5 kbp or more.
[10] The method according to [7], wherein the region between the upstream homology arm and the downstream homology arm of the donor DNA for recombination has a length of 5 kbp or more.
[11] The method according to [8], wherein the region between the upstream homology arm and the downstream homology arm of the donor DNA for recombination has a length of 8 kbp or more.
[12] A genome engineering kit for engineering two or more alleles in a chromosomal genome, comprising the following (i) and (ii):
   (i) a genome engineering system comprising a sequence-specific nucleic acid cleaving molecule capable of targeting a target region in the chromosomal genome and cleaving the target region, or a polynucleotide encoding the sequence-specific nucleic acid cleaving molecule, and
   (ii) two or more donor DNAs for selective markers, each of which has an upstream homology arm having a nucleotide sequence that can be homologously recombined with an upstream nucleotide sequence of the target region and a downstream homology arm having a nucleotide sequence that can be homologously recombined with a downstream nucleotide sequence of the target region, and comprises a nucleotide sequence of a selective marker gene between the upstream homology arm and the downstream homology arm, the two or more donor DNAs for selective markers respectively having distinguishably different selective marker genes, wherein the selective marker gene is unique to each type of donor DNA for the selective marker, and the number of types of the donor DNAs for selective markers is equal to or more than the number of the alleles that are subject to genome engineering.
[13] The kit according to [12], wherein the target region has a length of 5 kbp or more.
[14] The kit according to [13], wherein the target region has a length of 8 kbp or more.
[15] The kit according to any of [12] to [14], further comprising a donor DNA for recombination.
[16] The kit according to any of [12] to [15], wherein a region between an upstream homology arm and a downstream homology arm of the donor DNA for recombination has a length of 5 kbp or more.
[17] The kit according to any of [12] to [16], wherein a region between an upstream homology arm and a downstream homology arm of the donor DNA for recombination has a length of 8 kbp or more.
[18] The kit according to [12], wherein the target region has a length of 5 kbp or more, and a region between an upstream homology arm and a downstream homology arm of the donor DNA for recombination has a length of 5 kbp or more.
[19] The kit according to [18], wherein the target region has a length of 8 kbp or more, and a region between an upstream homology arm and a downstream homology arm of the donor DNA for recombination has a length of 8 kbp or more.
[20] The method according to [5], wherein the donor DNA for recombination has no nucleotide sequence in the region between the upstream homology arm and the downstream homology arm of the donor DNA for recombination, and the upstream and downstream sequences of the target region are seamlessly linked, without insertion, substitution and deletion of a base, in the thus-engineered two or more alleles in the chromosomal genome.
[21] The method according to [6] or [7], wherein the donor DNA for recombination has no nucleotide sequence in the region between the upstream homology arm and the downstream homology arm of the donor DNA for recombination, and the upstream and downstream sequences of the target region are seamlessly linked, without insertion, substitution and deletion of a base, in the thus-engineered two or more alleles in the chromosomal genome.
[22] The method according to [3], wherein a target sequence of site-specific recombinase is absent in the thus-engineered two or more alleles in the chromosomal genome.
[23] The method according to [4], wherein a target sequence of site-specific recombinase is absent in the thus-engineered two or more alleles in the chromosomal genome.
[24] The method according to [5], wherein a target sequence of site-specific recombinase is absent in the thus-engineered two or more alleles in the chromosomal genome.
[25] The method according to [6] or [7], wherein a target sequence of site-specific recombinase is absent in the thus-engineered two or more alleles in the chromosomal genome.
[26] The method according to any of [1] to [11] and [20] to [25], wherein in the step (b), single-cell cloning is not performed in a process up to the selection of the cell in which the two or more alleles are engineered.
[27] A cell having two or more alleles in the chromosomal genome in relation to a target region, wherein the respective target regions of the two or more alleles are deleted, and the upstream and downstream sequences of the target region are seamlessly linked without insertion, substitution and deletion of a base.
[28] The cell according to [27], wherein the target region has a length of 5 kbp or more.
[29] The cell according to [27] or [28], wherein the cell has no target sequence of site-specific recombinase in the genome.
[30] The method according to [6] or [7], wherein the region between the upstream homology arm and the downstream homology arm of the donor DNA for recombination has a length of 8 kbp or more.
[31] The kit according to [15], wherein the donor DNA for recombination has no nucleotide sequence in the region between the upstream homology arm and the downstream homology arm of the donor DNA for recombination.

### [Advantageous Effects of Invention]

According to the present invention, a genome engineering method and a genome engineering kit which are capable of engineering two or more alleles efficiently and engineering a relatively large region can be provided.

### [Brief Description of Drawings]

[Figure 1] Figure 1 shows the structures of donor DNAs prepared in Experimental Example 1.
[Figure 2] Figure 2 shows the step of introducing the donor DNAs prepared in Experimental Example 1 to cells. This figure also shows the designed positions of junction primers used for confirming the knock-in of the donor DNAs.
[Figure 3] Figure 3 shows results of performing junction PCR after knock-in of donor DNAs.
[Figure 4] Figure 4 shows the step of introducing donor DNAs prepared in Reference Experimental Example to cells. This figure also shows the designed positions of junction primers used for confirming the knock-in of the donor DNAs, and results of performing junction PCR after knock-in of the donor DNAs.
[Figure 5] Figure 5 shows the step of introducing a wild-type TP53 plasmid as a donor DNA to cells harboring the donor DNAs prepared in Experimental Example 1 (cells prepared in Experimental Example 2). This figure also shows the designed positions of primers used for confirming the knock-in of the wild-type TP53 plasmid.
[Figure 6] Figure 6 shows results of performing PCR using the primers of Figure 5 after knock-in of the wild-type TP53 plasmid.
[Figure 7] Figure 7 shows the step of introducing four types of donor DNAs prepared in Experimental Example 4 to cells prepared in Experimental Example 2. This figure also shows the designed positions of primers used for confirming the knock-in of the four types of donor DNAs.
[Figure 8] Figure 8 shows results of performing PCR using the primers of Figure 7 after knock-in of the four types of donor DNAs prepared in Experimental Example 4.
[Figure 9] Figure 9 shows results of conducting an experiment to induce deletion in both the alleles of TP53 gene in human iPS cells in Example 5.
[Figure 10] Figure 10 shows results of conducting an experiment to induce deletion in both the alleles of MLH1 gene in Example 6.
[Figure 11] Figure 11 shows results of conducting an experiment to induce deletion in both the alleles of CD44 gene in Example 6.
[Figure 12] Figure 12 shows results of conducting an experiment to induce deletion in both the alleles of MET gene in Example 6.
[Figure 13] Figure 13 shows results of conducting an experiment to induce deletion in both the alleles of APP gene in Example 6.
[Figure 14A] Figure 14A is a schematic view showing the induction of cleavage at two locations so as to sandwich a target region in a genome in the step of introducing donor DNAs for selective markers in the method of the present invention.
[Figure 14B] Figure 14B is a schematic view showing the induction of cleavage at one location near a target region in a genome in the step of introducing donor DNAs for selective markers in the method of the present invention.

### [Description of Embodiments]

### [Definition]

The terms "polynucleotide" and "nucleic acid" are used interchangeably with each other and each refer to a nucleotide polymer in which nucleotides are linked through phosphodiester bonds. The "polynucleotide" or the "nucleic acid" may be DNA, may be RNA, or may be constituted by a combination of DNA and RNA. The "polynucleotide" or the "nucleic acid" may be a polymer of natural nucleotides, may be a polymer of natural nucleotides and non-natural nucleotides (analogs of natural nucleotides, nucleotides modified at one of their base moiety, sugar moiety and phosphate moiety (e.g., phosphorothioate skeletons), etc.), or may be a polymer of non-natural nucleotides.

The nucleotide sequence of the "polynucleotide" or the "nucleic acid" is described by generally accepted single-letter codes unless otherwise specified. Each nucleotide sequence is described from the 5' side toward the 3' side unless otherwise specified. The nucleotide residues constituting the "polynucleotide" or the "nucleic acid" may be simply described by adenine, thymine, cytosine, guanine, or uracil, etc., or their single-letter codes.

The term "gene" refers to a polynucleotide containing at least one open reading frame encoding a particular protein. The gene can contain both an exon and an intron.

The terms "polypeptide", "peptide" and "protein" are used interchangeably with each other and each refer to a polymer of amino acids linked through amide bonds. The "polypeptide", the "peptide" or the "protein" may be a polymer of natural amino acids, may be a polymer of natural amino acids and non-natural amino acids (chemical analogs, modified derivatives, etc. of natural amino acids), or may be a polymer of non-natural amino acids. Each amino acid sequence is described from the N-terminal side toward the C-terminal side unless otherwise specified.

The term "alleles" refer to a set of nucleotide sequences present at the same locus on a chromosomal genome. In an embodiment, a diploid cell has two alleles at the same locus, and a triploid cell has three alleles at the same locus. In an embodiment, an additional allele may be formed by an abnormal copy of the chromosome or an abnormal additional copy of the locus.

The terms "genome engineering" and "genome editing" are used interchangeably with each other and each refer to mutagenesis at a desired position (target region) in the genome. The genome engineering can involve using a sequence-specific nucleic acid cleaving molecule designed so as to cleave DNA of the target region. In a preferred embodiment, the genome engineering can involve using nuclease engineered so as to cleave DNA of the target region. In a preferred embodiment, the genome engineering can involve using nuclease (e.g., TALEN or ZFN) engineered so as to cleave a target sequence having a particular nucleotide sequence in the target region. In a preferred embodiment, the genome engineering may employ sequence-specific endonuclease such as a restriction enzyme (e.g., meganuclease) having only one cleavage site in the genome (e.g., a restriction enzyme having 16-base sequence specificity (theoretically, which is present at a ratio of 1 out of 4¹⁶ bases), a restriction enzyme having 17-base sequence specificity (theoretically, which is present at a ratio of 1 out of 4¹⁷ bases), and a restriction enzyme having 18-base sequence specificity (theoretically, which is present at a ratio of 1 out of 4¹⁸ bases)) so as to cleave a target sequence having a particular nucleotide sequence in the target region. Typically, use of site-specific nuclease induces double-strand break (DSB) in DNA of the target region, followed by the repair of the genome by an endogenous process of cells, such as homology directed repair (HDR) and non-homologous end-joining repair (NHEJ). NHEJ is a repair method of linking ends that have undergone double-strand break, without the use of a donor DNA, and induces insertion and/or deletion (indel) with high frequency during the repair. HDR is a repair mechanism using a donor DNA and is also capable of introducing a desired mutation to a target region. Examples of the genome engineering technique preferably include a CRISPR/Cas system. The meganuclease that can be used is, for example, meganuclease selected from the group consisting of I-SceI, I-SceII, I-SceIII, I-SceIV, I-SceV, I-SceVI, I-SceVII, I-CeuI, I-CeuAIIP, I-CreI, I-CrepsbIP, I-CrepsbIIP, I-CrepsbIIIP, I-CrepsbIVP, I-TliI, I-PpoI, PI-PspI, F-SceI, F-SceII, F-SuvI, F-TevI, F-TevII, I-AmaI, I-AniI, I-ChuI, I-CmoeI, I-CpaI, I-CpaII, I-CsmI, I-CvuI, I-CvuAIP, I-DdiI, I-DdiII, I-DirI, I-DmoI, I-HmuI, I-HmuII, I-HsNIP, I-LlaI, I-MsoI, I-NaaI, I-NanI, I-NclIP, I-NgrIP, I-NitI, I-NjaI, I-Nsp236IP, I-PakI, I-PboIP, I-PcuIP, I-PcuAI, I-PcuVI, I-PgrIP, I-PobIP, I-PorI, I-PorIIP, I-PbpIP, I-SpBetaIP, I-ScaI, I-SexIP, I-SneIP, I-SpomI, I-SpomCP, I-SpomIP, I-SpomIIP, I-SquIP, I-Ssp68031, I-SthPhiJP, I-SthPhiST3P, I-SthPhiSTe3bP, I-TdeIP, I-TevI, I-TevII, I-TevIII, I-UarAP, I-UarHGPAIP, I-UarHGPA13P, I-VinIP, I-ZbiIP, PI-Mtul, PI-MtuHIP, PI-MtuHIIP, PI-PfuI, PI-PfuII, PI-PkoI, PI-PkoII, PI-Rma43812IP, PI-SpBetaIP, PI-SceI, PI-TfuI, PI-TfuII, PI-ThyI, PI-TliI, and PI-TliII and their functional derivative restriction enzymes, and a cleavage site (or a recognition site) thereof, preferably meganuclease which is a restriction enzyme having 18-base or more sequence specificity, and a cleavage site (or a recognition site) thereof, particularly, meganuclease that does not cleave one location or two or more locations of the genome in a cell, or a cleavage site thereof.

The term "target region" refers to a genomic region that is subject to genome engineering.

The term "donor DNA" is DNA for use in the repair of double-strand break of DNA and refers to DNA that can be homologously recombined with neighboring DNA of a target region. The donor DNA comprises, as homology arms, a nucleotide sequence upstream and a nucleotide sequence downstream of a target region (e.g., nucleotide sequences adjacent to a target region). In the present specification, the homology arm consisting of an upstream nucleotide sequence of (e.g., an upstream nucleotide sequence adjacent to) the target region is also referred to as an "upstream homology arm", and the homology arm consisting of a downstream nucleotide sequence of (e.g., a downstream nucleotide sequence adjacent to) the target sequence is also referred to as a "downstream homology arm". The donor DNA can comprise a desired nucleotide sequence between the upstream homology arm and the downstream homology arm. The length of each homology arm is preferably 300 bp or more and is usually on the order of 500 to 3000 bp. The upstream homology arm and the downstream homology arm may have the same lengths or may have different lengths. When a target region successfully induces homologous recombination with the donor DNA after sequence-dependent cleavage, the sequence between the nucleotide sequence upstream and the nucleotide sequence downstream of the target region is replaced with the sequence of the donor DNA.

The term "upstream" of the target region means a DNA region positioned on the 5' side of a reference nucleotide strand in the double-stranded DNA of the target region. The term "downstream" of the target region means a DNA region positioned on the 3' side of the reference nucleotide strand. When the target region comprises a protein coding sequence, the reference nucleotide strand is usually a sense strand. In general, a promoter is positioned upstream of the protein coding sequence. A terminator is positioned downstream of the protein coding sequence.

The term "sequence-specific nucleic acid cleaving molecule" refers to a molecule that can recognize a particular nucleic acid sequence and cleave a nucleic acid at the particular nucleic acid sequence. The sequence-specific nucleic acid cleaving molecule is a molecule having activity of cleaving a nucleic acid in a sequence-specific manner (sequence-specific nucleic acid cleaving activity).

The term "target sequence" refers to a DNA sequence, in the genome, to be cleaved by the sequence-specific nucleic acid cleaving molecule. When the sequence-specific nucleic acid cleaving molecule is Cas protein, the target sequence refers to a DNA sequence, in the genome, to be cleaved by the Cas protein. In the case of using Cas9 protein as the Cas protein, the target sequence needs to be a sequence adjacent to the 5' side of a protospacer adjacent motif (PAM). The target sequence is usually selected as a sequence of 17 to 30 bases (preferably 18 to 25 bases, more preferably 19 to 22 bases, further preferably 20 bases) immediately adjacent to the 5' side of PAM. The target sequence can be designed using a design tool known in the art such as CRISPR DESIGN (crispr.mit.edu/).

The term "Cas protein" refers to CRISPR-associated protein. In a preferred embodiment, the Cas protein forms a complex with guide RNA and exhibits endonuclease activity or nickase activity. Cas proteins include, for example, but not particularly limited to, Cas9 protein, Cpf1 protein, C2c1 protein, C2c2 protein, and C2c3 protein. The Cas protein encompasses wild-type Cas protein and its homologs (paralogs and orthologs), and their mutants as long as they exhibit endonuclease activity or nickase activity in cooperation with guide RNA.

In a preferred embodiment, the Cas protein is involved in a class 2 CRISPR/Cas system and more preferably involved in a type II CRISPR/Cas system. Preferred examples of the Cas protein include Cas9 protein.

The term "Cas9 protein" refers to Cas protein that is involved in a type II CRISPR/Cas system. The Cas9 protein forms a complex with guide RNA and exhibits activity of cleaving DNA of a target region in cooperation with the guide RNA. The Cas9 protein encompasses wild-type Cas9 protein and its homologs (paralogs and orthologs), and their mutants as long as they exhibit the activity described above. The wild-type Cas9 protein has a RuvC domain and a HNH domain as nuclease domains. In the present specification, any one of the RuvC domain and the HNH domain in the Cas9 protein may be inactivated. Cas9 in which any one of the RuvC domain and the HNH domain is inactivated introduces single-strand cleavage (nick) in double-stranded DNA. Hence, in the case of using Cas9 in which any one of the RuvC domain and the HNH domain is inactivated in the cleavage of double-stranded DNA, an engineering system can be configured such that a target sequence of Cas9 is set in each of the sense strand and the antisense strand and nick for the sense strand and nick for the antisense strand occur at sufficiently close positions, thereby inducing double-strand break.

Examples of the organism species from which the Cas9 protein is derived preferably include, but are not particularly limited to, bacteria belonging to the genus *Streptococcus,* the genus *Staphylococcus,* the genus *Neisseria,* or the genus *Treponema.* More specifically, examples thereof preferably include Cas9 protein derived from *S. pyogenes, S. thermophilus, S. aureus, N. meningitidis,* or *T. denticola.* In a preferred embodiment, the Cas9 protein is *S. pyogenes*-derived Cas9 protein.

Information on the amino acid sequence of each Cas protein, and its coding sequence can be obtained from various databases such as GenBank, UniProt, and Addgene. For example, a sequence registered under plasmid No. 42230 in Addgene can be used as the amino acid sequence of *S. pyogenes* Cas9 protein. One example of the amino acid sequence of *S. pyogenes* Cas9 protein is shown in SEQ ID NO: 1.

The terms "guide RNA" and "gRNA" are used interchangeably with each other and each refer to RNA that can form a complex with Cas protein and lead the Cas protein to a target region. In a preferred embodiment, the guide RNA comprises CRISPR RNA (crRNA) and transactivating CRISPR RNA (tracrRNA). crRNA is involved in binding to a target region in the genome, and tracrRNA is involved in binding to the Cas protein. In a preferred embodiment, crRNA comprises a spacer sequence and a repeat sequence, and the spacer sequence binds to a complementary strand of a target sequence in the target region. In a preferred embodiment, tracrRNA comprises an anti-repeat sequence and a 3' tail sequence. The anti-repeat sequence has a sequence complementary to the repeat sequence of crRNA and forms base pairs with the repeat sequence. The 3' tail sequence usually forms three stem loops.

The guide RNA may be single-guide RNA (sgRNA) in which the 5' end of tracrRNA is linked to the 3' end of crRNA, or may be formed by the base pairing of the repeat sequence and the anti-repeat sequence of crRNA and tracrRNA prepared as separate RNA molecules. In a preferred embodiment, the guide RNA is sgRNA.

The repeat sequence of crRNA and the sequence of tracrRNA can be appropriately selected according to the type of the Cas protein, and sequences derived from the same bacterial species as that for the Cas protein can be used.

In the case of using, for example, *S. pyogenes-*derived Cas9 protein, the length of sgRNA can be about 50 to 220 nucleotides (nt) and is preferably about 60 to 180 nt, more preferably about 80 to 120 nt. The length of crRNA can be a length of about 25 to 70 bases including a spacer sequence, and is preferably about 25 to 50 nt. The length of tracrRNA can be about 10 to 130 nt and is preferably about 30 to 80 nt.

The repeat sequence of crRNA may be the same as that in the bacterial species from which the Cas protein is derived, or may be 3'-terminally truncated. tracrRNA may have the same sequence as that of mature tracrRNA in the bacterial species from which the Cas protein is derived, or may be a terminally truncated form of the mature tracrRNA obtained by the cleavage of the 5' end and/or the 3' end. For example, tracrRNA can be a terminally truncated form of the mature tracrRNA obtained by the removal of approximately 1 to 40 nucleotide residues from the 3' end. tracrRNA can be a terminally truncated from of the mature tracrRNA obtained by the removal of approximately 1 to 80 nucleotide residues from the 5' end. tracrRNA can be, for example, a terminally truncated from of the mature tracrRNA obtained by the removal of approximately 1 to 20 nucleotide residues from the 5' end and the removal of approximately 1 to 40 nucleotide residues from the 3' end.

Various crRNA repeat sequences and tracrRNA sequences for sgRNA design have been proposed. Those skilled in the art can design sgRNA on the basis of a technique known in the art (e.g., Jinek et al., (2012) Science, 337, 816-21; Mali et al., (2013) Science, 339: 6121, 823-6; Cong et al., (2013) Science, 339: 6121, 819-23; Hwang et al., (2013) Nat. Biotechnol. 31: 3, 227-9; and Jinek et al., (2013) eLife, 2, e00471).

The terms "protospacer adjacent motif" and "PAM" are used interchangeably with each other and each refer to a sequence that is recognized by Cas protein upon DNA cleavage by the Cas protein. The sequence and position of PAM differ depending on the type of the Cas protein. In the case of, for example, Cas9 protein, PAM needs to be a downstream sequence immediately adjacent to the 3' side of the target sequence. The sequence of PAM compatible with the Cas9 protein differs depending on the bacterial species from which the Cas9 protein is derived. For example, PAM compatible with *S. pyogenes* Cas9 protein is "NGG". PAM compatible with *S. thermophilus* Cas9 protein is "NNAGAA". PAM compatible with *S. aureus* Cas9 protein is "NNGRRT" or "NNGRR(N)". PAM compatible with *N. meningitidis* Cas9 protein is "NNNNGATT". PAM compatible with *T. denticola* Cas9 protein is "NAAAAC" ("R" is A or G; "N" is A, T, G or C) .

The terms "spacer sequence" and "guide sequence" are used interchangeably with each other and each refer to a sequence that is contained in guide RNA and is capable of binding to a complementary strand of a target sequence. Usually, the spacer sequence is a sequence identical to the target sequence (except that T in the target sequence corresponds to U in the spacer sequence). In an embodiment of the present invention, the spacer sequence can contain a 1-base mismatch or mismatches of two or more bases against the target sequence. In the case of containing mismatches of two or more bases, the mismatches may be present at adjacent positions or may be present at distant positions. In a preferred embodiment, the spacer sequence can contain 1- to 5-base mismatches against the target sequence. In a particularly preferred embodiment, the spacer sequence may contain a 1-base mismatch against the target sequence.

In the guide RNA, the spacer sequence is placed on the 5' side of crRNA.

The term "operably linked" used in relation to a polynucleotide means that a first nucleotide sequence is placed sufficiently close to a second nucleotide sequence so that the first nucleotide sequence is capable of influencing the second nucleotide sequence or a region controlled by the second nucleotide sequence. For example, the phrase "polynucleotide is operably linked to a promoter" means that the polynucleotide is linked so as to be expressed under the control of the promoter.

The term "expressible state" refers to a state in which a polynucleotide can be transcribed in a cell having the introduced polynucleotide.

The term "expression vector" is a vector containing a subject polynucleotide and refers to a vector having a system that puts the subject polynucleotide in an expressible state in a cell having the introduced vector. For example, the "Cas protein expression vector" means a vector that permits expression of the Cas protein in a cell having the introduced vector. For example, the "guide RNA expression vector" means a vector that permits expression of the guide RNA in a cell having the introduced vector.

In the present specification, the sequence identity (or homology) between nucleotide sequences or amino acid sequences is determined as the ratio of identical bases or amino acids to the whole nucleotide sequences or the whole amino acid sequences, except for gaps, in alignments obtained by juxtaposing two nucleotide sequences or amino acid sequences so as to attain the highest identity of the corresponding bases or amino acids while placing the gaps in moieties corresponding to insertion and deletion. The sequence identity between nucleotide sequences or amino acid sequences can be determined using various homology search software known in the art. For example, the value of sequence identity between nucleotide sequences can be obtained by calculation based on alignments obtained with homology search software BLASTN known in the art, and the value of sequence identity between amino acid sequences can be obtained by calculation based on alignments obtained with homology search software BLASTP known in the art.

### [Genome engineering method]

In one embodiment, the present invention provides a genome engineering method for engineering two or more alleles in a chromosomal genome. The genome engineering method comprises the following steps (a) and (b):
(a) introducing the following (i) and (ii) to a cell comprising the chromosome:
   (i) a genome engineering system comprising a sequence-specific nucleic acid cleaving molecule targeting a target region in the chromosomal genome, or a polynucleotide encoding the sequence-specific nucleic acid cleaving molecule, and
   (ii) two or more donor DNAs for selective markers, each of which comprises a nucleotide sequence of a selective marker gene between an upstream homology arm having a nucleotide sequence homologous to an upstream nucleotide sequence adjacent to the target region and a downstream homology arm having a nucleotide sequence homologous to a downstream nucleotide sequence adjacent to the target region, the two or more donor DNAs for selective markers respectively having different selective marker genes, wherein the number of types of the donor DNAs for selective markers is equal to or more than the number of the alleles that are subject to genome engineering; and
(b) after the step (a), selecting the cell on the basis of all the selective marker genes carried by the two or more donor DNAs for selective markers. In this embodiment, the selective marker gene can be unique to each type of donor DNA for the selective marker. In this embodiment, the step (b) can be the step of, after the step (a), respectively homologously recombining the two or more alleles with different types of donor DNAs for selective markers so that the distinguishably different unique selective marker genes are respectively introduced in the two or more alleles, and selecting a cell expressing all the distinguishably different selective marker genes thus introduced (step for positive selection). The method may be a method for preparing a cell in which two or more alleles in the chromosomal genome are engineered.

In one embodiment, the present invention can be a method for preparing a cell in which two or more alleles in the chromosomal genome are engineered, comprising the steps of:
(a) introducing the following (i) and (ii) to a cell comprising two or more alleles to introduce a selective marker gene to each of the two or more alleles:
   (i) a genome engineering system comprising a sequence-specific nucleic acid cleaving molecule capable of targeting a target region in the two or more alleles in the chromosomal genome and cleaving the target region, or a polynucleotide encoding the sequence-specific nucleic acid cleaving molecule, and
   (ii) two or more donor DNAs for selective markers, each of which has an upstream homology arm having a nucleotide sequence that can be homologously recombined with an upstream nucleotide sequence of the target region and a downstream homology arm having a nucleotide sequence that can be homologously recombined with a downstream nucleotide sequence of the target region, and comprises a nucleotide sequence of the selective marker gene between the upstream homology arm and the downstream homology arm, the two or more donor DNAs for selective markers respectively having distinguishably different selective marker genes, wherein the selective marker gene is unique to each type of donor DNA for the selective marker, and the number of types of the donor DNAs for selective markers is equal to or more than the number of the alleles that are subject to genome engineering; and
(b) after the step (a), selecting a cell having the distinguishably different unique selective marker genes, which are respectively introduced in the two or more alleles by respectively homologously recombining the two or more alleles with different types of donor DNAs for selective markers, the cell expressing all the distinguishably different selective marker genes thus introduced (step for positive selection).

### (Step (a))

In the step (a), (i) and (ii) described above are introduced to a cell comprising the chromosome.

The cell for use in the genome engineering method of the present embodiment is not particularly limited and can be a cell having a diploid or higher chromosomal genome. The cell may be a diploid, may be a triploid, or may be a tetraploid or higher. Examples of the cell include, but are not particularly limited to, eukaryotic cells. The cell may be a plant cell, may be an animal cell, or may be a fungal cell. The animal cell is not particularly limited and may be any cell of humans, nonhuman mammals, birds, reptiles, amphibians, fish, insects, and other invertebrate animals. The cell for use in the genome engineering method of the present embodiment is not a cell having no allele (e.g., a cell having a monoploid chromosomal genome, for example, a prokaryotic cell).

The target region that is subject to genome engineering can be an arbitrary region, in the genome, having one or more alleles. The size of the target region is not particularly limited. The genome engineering method of the present embodiment can engineer a region having a size larger than ever. The target region may be, for example, 10 kbp or more. The target region may be, for example, 100 bp or more, 200 bp or more, 400 bp or more, 800 bp or more, 1 kbp or more, 2 kbp or more, 3 kbp or more, 4 kbp or more, 5 kbp or more, 8 kbp or more, 10 kbp or more, 20 kbp or more, 40 kbp or more, 80 kbp or more, 100 kbp or more, 200 kbp or more, 300 kbp or more, or 400 kbp or more. The target region can be, for example, a region containing one to several genes, a region containing one gene, or a partial region of one gene. In an embodiment, the engineered cell lacks the target region.

### <(i) Genome engineering system>

The "genome engineering system" means a molecular mechanism capable of engineering a desired target region. The genome engineering system comprises a sequence-specific nucleic acid cleaving molecule targeting a target region in a chromosomal genome, or a polynucleotide encoding the sequence-specific nucleic acid cleaving molecule.

The sequence-specific nucleic acid cleaving molecule is not particularly limited as long as the molecule has sequence-specific nucleic acid cleaving activity. The sequence-specific nucleic acid cleaving molecule may be a synthetic organic compound or may be a biopolymer compound such as a protein. Examples of the synthetic organic compound having sequence-specific nucleic acid cleaving activity include pyrrole-imidazole-polyamide. Examples of the protein having sequence-specific site cleaving activity include sequence-specific endonuclease.

The sequence-specific endonuclease is an enzyme that can cleave a nucleic acid at a predetermined sequence. The sequence-specific endonuclease can cleave double-stranded DNA at a predetermined sequence. The sequence-specific endonuclease is not particularly limited. Examples thereof include, but are not limited to, zinc finger nuclease (ZFN)), TALEN (transcription activator-like effector nuclease), and Cas protein.

ZFN is artificial nuclease containing a nucleic acid cleavage domain conjugated with a binding domain containing a zinc finger array. Examples of the cleavage domain include the cleavage domain of type II restriction enzyme FokI. Zinc finger nuclease capable of cleaving a target sequence can be designed by a method known in the art.

TALEN is artificial nuclease containing a DNA cleavage domain (e.g., a FokI domain) as well as the DNA binding domain of a transcription activator-like (TAL) effector. A TALE construct capable of cleaving a target sequence can be designed by a method known in the art (e.g., Zhang, Feng et. al. (2011) Nature Biotechnology 29 (2)).

In the case of using Cas protein as the sequence-specific nucleic acid cleaving molecule, the genome engineering system comprises a CRISPR/Cas system. Specifically, the genome engineering system preferably comprises Cas protein, and guide RNA having a nucleotide sequence homologous to a nucleotide sequence within the target region. The guide RNA can comprise, as a spacer sequence, a sequence homologous to a sequence (target sequence) within the target region. The guide RNA can bind to DNA within the target region and does not have to have a sequence completely identical to the target sequence. This binding can be formed under physiological conditions in the cell nucleus. The guide RNA can contain, for example, 0- to 3-base mismatches with respect to the target sequence. The number of the mismatches is preferably 0 to 2 bases, more preferably 0 to 1 bases, further preferably zero mismatch. The guide RNA can be designed on the basis of a method known in the art. The genome engineering system is preferably a CRISPR/Cas system and preferably comprises Cas protein and guide RNA. The Cas protein is preferably Cas9 protein.

The sequence-specific endonuclease may be introduced as a protein to the cell, or may be introduced as a polynucleotide encoding the sequence-specific endonuclease to the cell. For example, mRNA of the sequence-specific endonuclease may be introduced, or an expression vector of the sequence-specific endonuclease may be introduced. In the expression vector, a coding sequence of the sequence-specific endonuclease (sequence-specific endonuclease gene) is operably linked to a promoter. The promoter is not particularly limited, and, for example, various pol II promoters can be used. Examples of the pol II promoter include, but are not particularly limited to, CMV promoter, EF1 promoter (EF1α promoter), SV40 promoter, MSCV promoter, hTERT promoter, β actin promoter, CAG promoter, and CBh promoter.

The promoter may be an inducible promoter. The inducible promoter is a promoter that can induce the expression of a polynucleotide operably linked to this promoter only in the presence of an inducer that drives the promoter. Examples of the inducible promoter include promoters, such as heat shock promoter, which induce gene expression by heating. Examples of the inducible promoter further include promoters for which the inducer that drives the promoter is a drug. Examples of such a drug-inducible promoter include Cumate operator sequences, λ operator sequences (e.g., 12 × λOp), and tetracycline-inducible promoter. Examples of the tetracycline-inducible promoter include promoters that drive gene expression in the presence of tetracycline or a derivative thereof (e.g., doxycycline), or reverse tetracycline-controlled transactivator (rtTA). Examples of the tetracycline-inducible promoter include TRE3G promoter.

An expression vector known in the art can be used without particular limitations. Examples of the expression vector include plasmid vectors and virus vectors. When the sequence-specific endonuclease is Cas protein, the expression vector may contain a coding sequence of the Cas protein (Cas protein gene) as well as a guide RNA coding sequence (guide RNA gene). In this case, it is preferred that the guide RNA coding sequence (guide RNA gene) should be operably linked to pol III promoter. Examples of the pol III promoter include mouse and human U6-snRNA promoters, human H1-RNase P RNA promoter, and human valine-tRNA promoter.

### <(ii) Donor DNAs for selective markers>

The donor DNAs for selective markers are donor DNAs for knocking-in selective markers to target regions. Each of the donor DNAs for selective markers comprises the nucleotide sequences of one or more selective marker genes between an upstream homology arm having a nucleotide sequence homologous to an upstream nucleotide sequence adjacent to the target region and a downstream homology arm having a nucleotide sequence homologous to a downstream nucleotide sequence adjacent to the target region.

The donor DNAs for selective markers can have a length of, for example, but not particularly limited to, 1 kb or more, 2 kb or more, 3 kb or more, 4 kb or more, 5 kb or more, 6 kb or more, 7 kb or more, 8 kb or more, 9 kb or more, 9.5 kb or more, or 10 kb or more. The donor DNAs for selective markers can have a length of, for example, but not particularly limited to, 50 kb or less, 45 kb or less, 40 kb or less, 35 kb or less, 30 kb or less, 25 kb or less, 20 kb or less, 15 kb or less, 14 kb or less, 13 kb or less, 12 kb or less, 11 kb or less, 10 kb or less, 9 kb or less, 8 kb or less, 7 kb or less, 6 kb or less, 5 kb or less, or 4 kb or less.

The "selective marker" means a protein that permits cells to be selected on the basis of the presence or absence of its expression. The selective marker gene is a gene encoding the selective marker. In the case of selecting selective marker-expressing cells from a cell population in which the selective marker-expressing cells coexist with selective marker non-expressing cells, the selective marker is referred to as a "positive selective marker" or a "selective marker for positive selection". In the case of selecting selective marker non-expressing cells from a cell population in which selective marker-expressing cells coexist with the selective marker non-expressing cells, the selective marker is referred to as a "negative selective marker" or a "selective marker for negative selection". Different selective markers mean that the selective markers can be distinguished from each other (e.g., distinguishably different), and means that, for example, the selective markers can be distinguished from each other at least in terms of physiological properties such as drug resistance property or other physicochemical properties imparted to cells harboring the selective markers. Specifically, different selective markers mean that one of a plurality of different selective markers can be detected distinguishably from the other selective marker(s), or permits drug selection distinguishably from the other selective marker(s). The phrase "selective marker gene is unique to each type of donor DNA for the selective marker" means that the selective marker gene carried by one of the donor DNAs for selective markers is not contained in the other type(s) of donor DNA(s) for selective marker(s), or the selective marker gene, if contained in plural types of donor DNAs, is configured such that this selective marker gene is not expressed from two or more types of donor DNAs at the same time. In this respect, the two or more types of donor DNAs may be the same with each other except for their respective selective markers, or may differ in sequence and/or configuration other than the selective markers.

The positive selective marker is not particularly limited as long as a cell expressing the positive selective marker can be selected. Examples of the positive selective marker gene include drug resistance genes, fluorescent protein genes, luminescent enzyme genes, and chromogenic enzyme genes.

The negative selective marker is not particularly limited as long as a cell not expressing the negative selective marker can be selected. Examples of the negative selective marker gene include suicide genes (thymidine kinase, etc.), fluorescent protein genes, luminescent enzyme genes, and chromogenic enzyme genes. When the negative selective marker gene is a gene that has negative influence on the survival of cells (e.g., a suicide gene), the negative selective marker gene can be operably linked to an inducible promoter. The negative selective marker gene thus operably linked to the inducible promoter can be expressed only when the removal of cells having the negative selective marker gene is desired. The negative selective marker gene, for example, an optically detectable (e.g., fluorescent, luminescent, and chromogenic) marker gene (visible marker gene), may be constitutively expressed because of having little negative influence on the survival of cells.

Examples of the drug resistance gene include, but are not limited to, puromycin resistance gene, blasticidin resistance gene, geneticin resistance gene, neomycin resistance gene, tetracycline resistance gene, kanamycin resistance gene, zeocin resistance gene, hygromycin resistance gene, and chloramphenicol resistance gene.

Examples of the fluorescent protein gene include, but are not limited to, green fluorescent protein (GFP) gene, yellow fluorescent protein (YFP) gene, and red fluorescent protein (RFP) gene.

Examples of the luminescent enzyme gene include, but are not limited to, luciferase gene.

Examples of the chromogenic enzyme gene include, but are not limited to, β galactosidase gene, β glucuronidase gene, and alkaline phosphatase gene.

Examples of the suicide gene include, but are not limited to, herpes simplex virus thymidine kinase (HSV-TK) and inducible caspase 9.

The selective marker genes carried by the donor DNAs for selective markers are preferably positive selective marker genes. Specifically, cells expressing the selective markers can be selected as cells in which the selective marker genes are knocked in.

The upstream homology arm has a nucleotide sequence that can be homologously recombined with an upstream nucleotide sequence of the target region in the genome to be engineered, and has, for example, a nucleotide sequence homologous to an upstream nucleotide sequence adjacent to the target sequence. The downstream homology arm has a nucleotide sequence that can be homologously recombined with a downstream nucleotide sequence of the target region in the genome to be engineered, and has, for example, a nucleotide sequence homologous to a downstream nucleotide sequence adjacent to the target sequence. The upstream homology arm and the downstream homology arm are not particularly limited by their lengths and sequences as long as these homology arms are that can be homologously recombined with the neighboring regions of the target region. The upstream homology arm and the downstream homology arm are not necessarily required to be identical to the upstream sequence and the downstream sequence, respectively, of the target region as long as these homology arms are that can be homologously recombined therewith. For example, the upstream homology arm can be a sequence having 90% or more sequence identity (homology) to the upstream nucleotide sequence adjacent to the target region and preferably has 92% or more, 93% or more, 94% or more, 95% or more, 96% or more, 97% or more, 98% or more, or 99% or more sequence identity thereto. For example, the downstream homology arm can be a sequence having 90% or more sequence identity (homology) to the downstream nucleotide sequence adjacent to the target region and preferably has 92% or more, 93% or more, 94% or more, 95% or more, 96% or more, 97% or more, 98% or more, or 99% or more sequence identity thereto. The engineering efficiency of the alleles can be enhanced provided that at least any one of the upstream homology arm and the downstream homology arm is located closer to a cleavage location in or near the target region. In this context, the term "close" can mean that the distance between two sequences is 100 bp or less, 50 bp or less, 40 bp or less, 30 bp or less, 20 bp or less, or 10 bp or less. In an embodiment, the cleavage is made at one location. In an embodiment, the cleavage is made at two or more locations. In the case of introducing cleavage at a plurality of locations in the genome, one location of the cleavage can be set upstream of the target region and another location can be set downstream of the target region. When the whole target region or almost the whole target region is excised from the genome by cleavage at two locations in the presence of the donor DNAs for selective markers, the donor DNAs for selective markers cause homology directed repair with the upstream and downstream of the target region so that the deleted regions are replaced with the sequences of the donor DNAs for selective markers. In this way, the efficiency of recombination can be enhanced as compared with the case of making cleavage at one location. The engineering efficiency of the alleles can be more enhanced provided that at least any one, preferably both, of the upstream homology arm and the downstream homology arm is(are) located closer to a cleavage location in or near the target region.

In the donor DNAs for selective markers, the selective marker gene is positioned between the upstream homology arm and the downstream homology arm. As a result, in the case of introducing the donor DNAs for selective markers, together with the genome engineering system (i), to the cell, the selective marker gene is introduced to the target region by HDR (this is referred to gene knock-out when the gene is disrupted, and referred to as gene knock-in when the desired gene is introduced; a gene may be knocked out while another gene can be knocked in).

It is preferred that the selective marker gene should be operably linked to a promoter so as to be expressed under the control of an appropriate promoter. The promoter can be appropriately selected according to the type of the cell to which the donor DNAs are to be introduced. Examples of the promoter include SRα promoter, SV40 early promoter, retrovirus LTR, CMV (cytomegalovirus) promoter, RSV (Rous sarcoma virus) promoter, HSV-TK (herpes simplex virus thymidine kinase) promoter, EF1α promoter, metallothionein promoter, and heat shock promoter. Each of the donor DNAs for selective markers may have, for example, an arbitrary control sequence such as an enhancer, a poly-A addition signal, or a terminator.

Each of the donor DNAs for selective markers may have an insulator sequence. The "insulator" refers to a sequence that ensures or enhances the independence of transcriptional regulation of DNA flanked by its regions by blocking or mitigating the influence of adjacent chromosomal environments. The insulator is defined by an enhancer blocking effect (effect of blocking the influence of an enhancer on promoter activity by the insulator inserted between the enhancer and the promoter), and a suppressive effect on a position effect (effect of preventing the expression of a transgene from being influenced by the position of the insert in the genome, by the insulators flanking both sides of the transgene). Each of the donor DNAs for selective markers may have an insulator sequence between the upstream arm and the selective marker gene (or between the upstream arm and a promoter that controls the selective marker gene). Each of the donor DNAs for selective markers may have an insulator sequence between the downstream arm and the selective marker gene.

The donor DNAs for selective markers may be linear or may be cyclic and are preferably cyclic. Preferably, the donor DNAs for selective markers are plasmids. Each of the donor DNAs for selective markers may comprise an arbitrary sequence in addition to the sequences described above. For example, a spacer sequence may be contained wholly or partially between the respective sequences of the upstream homology arm, the insulator, the selective marker gene, and the downstream homology arm.

In the step (a), the donor DNAs for selective markers, the number of types of which is equal to or more than the number of the alleles that are subject to genome engineering, are introduced to the cell. Such different types of donor DNAs for selective markers respectively have different (distinguishable) types of selective marker genes. In an embodiment, the different types of donor DNAs for selective markers do not have completely identical selective marker genes or sets thereof. In short, the first type of donor DNA for a selective marker has the first type of selective marker gene, and the second type of donor DNA for a selective marker has the second type of selective marker gene. The third type of donor DNA for a selective marker has the third type of selective marker gene. The same holds true for subsequent types of donor DNAs for selective markers. When the number of the alleles that are subject to genome engineering is 2, the types of the donor DNAs for selective markers are two or more types. When the number of the alleles that are subject to genome engineering is 3, the types of the donor DNAs for selective markers are three or more types. In an embodiment, one donor DNA for selective markers may have two or more types of different (distinguishable) selective markers (in this case as well, the different types of donor DNAs for selective markers must respectively have different (distinguishable) types of (e.g., unique) selective marker genes). In an embodiment, the donor DNAs for selective markers do not have a recombination sequence of site-specific recombinase (e.g., a loxP sequence and its variant which are recombined by Cre recombinase). In an embodiment, the method of the present invention employs neither site-specific recombinase nor its recombination sequence (e.g., a loxP sequence and its variant which are recombined by Cre recombinase). When site-specific recombinase is used, one recombination sequence of the site-specific recombinase usually remains in the genome after editing. By contrast, in an embodiment, the engineered genome of the cell obtained by the method of the present invention does not have a recombination sequence (which is a foreign sequence) of site-specific recombinase.

The number of types of the donor DNAs for selective markers can be equal to or more than the number of the alleles that are subject to genome engineering, and the upper limit is not particularly limited. Use of the donor DNAs for selective markers, the number of types of which is equal to or more than the number of the alleles that are subject to genome engineering, enables the two or more alleles to be stably engineered. The number of types of the donor DNAs for selective markers is preferably equal to or more by approximately 1 or 2 than the number of the alleles that are subject to genome engineering, more preferably equal to the number of the alleles that are subject to genome engineering, from the viewpoint of a selection operation in the step (b) mentioned later.

A method for introducing (i) and (ii) described above to the cell is not particularly limited, and a method known in the art can be used without particular limitations. Examples of the method for introducing (i) and (ii) to the cell include, but are not limited to, viral infection method, lipofection method, microinjection method, calcium phosphate method, DEAE-dextran method, electroporation method, and particle gun method. As a result of introducing (i) and (ii) described above to the cell, DNA of the target region is cleaved by the sequence-specific nucleic acid cleaving molecule of said (i), followed by the knock-in of each of the selective markers of the donor DNAs for selective markers of said (ii) in the target region by HDR. In this respect, when the two or more donor DNAs for selective markers have the same upstream homology arms and downstream homology arms, the donor DNAs can be randomly knocked in the two or more alleles of the target regions. However, the two or more donor DNAs for selective markers do not have to have completely identical nucleotide sequences of the homology arms because the donor DNAs can respectively engineer the two or more alleles as long as the homology arms have nucleotide sequences that can be homologously recombined with upstream sequences and downstream sequences of the respective target regions of the two or more alleles. In an embodiment, the nucleotide sequences of the upstream and downstream homology arms in the two or more donor DNAs for selective markers may have nucleotide sequences having higher identity to the upstream sequences and downstream sequences of the target regions of their respective alleles (e.g., the nucleotide sequences may be optimized in that way).

In an embodiment, each of the donor DNAs for selective markers has an upstream homology arm and a downstream homology arm and has a selective marker gene between the upstream homology arm and the downstream homology arm, and preferably, may further have a target sequence of endonuclease (nucleotide sequence-specific nucleic acid cleaving molecule), such as a cleavage site of meganuclease. In a preferred embodiment of this embodiment, the selective markers include selective marker genes for positive selections and a marker gene for negative selection. In another preferred embodiment, the selective markers include selective markers for positive selections but may not include a negative selective marker gene aside therefrom. In a preferred embodiment, the selective marker gene for positive selection may also be used for negative selection. Examples of such a marker gene include visible marker genes.

A set of two or more donor DNAs for selective markers is a combination of the donor DNAs for selective markers described above, and these donor DNAs respectively have selective marker genes for positive selections distinguishable from each other. In the set, each of the donor DNAs may further have a target sequence of endonuclease (nucleotide sequence-specific nucleic acid cleaving molecule), such as a cleavage site of meganuclease. Their respective target sequences may be different from each other and are preferably identical (or can be cleaved by the same nucleotide sequence-specific nucleic acid cleaving molecule). The length of the donor DNAs for selective markers is as described above and can be, for example, 5 kbp or more, 8 kbp or more, or 10 kbp or more.

### (Step (b))

After the step (a), the step (b) is performed. In the step (b), a cell in which distinguishably different selective marker genes or a combination thereof are respectively introduced in the two or more alleles is selected on the basis of the expression of the distinguishably different selective marker genes. More specifically, in the step (b), the two or more alleles are respectively homologously recombined with different types of donor DNAs for selective markers so that the distinguishably different unique selective marker genes are respectively introduced in the two or more alleles, and a cell expressing all the distinguishably different selective marker genes thus introduced is selected. In an embodiment, in the step (b), a cell in which these alleles are engineered by the introduction of the donor DNAs for different selective markers is selected on the basis of the expression of all the selective marker genes, integrated in the chromosomal genome, as the selective marker genes carried by the two or more donor DNAs for selective markers. In an embodiment, in the step (b), a cell is selected on the basis of all the selective marker genes carried by the two or more donor DNAs for selective markers. In an embodiment, in the step (b), a cell in which these alleles are engineered by the introduction of the donor DNAs for distinguishable selective markers is selected on the basis of the expression of all the selective marker genes (marker genes for positive selections), integrated in the chromosomal genome, as the selective marker genes carried by the two or more donor DNAs for selective markers. In an embodiment, the alleles in the cell obtained in the step (b) respectively have different marker genes for positive selections. In an embodiment, the alleles in the cell obtained in the step (b) may respectively have common marker genes for positive selections. In an embodiment, in the step (b), single-cell cloning is not performed {however, single-cell cloning to be performed after selection of the cell in which the two or more alleles are engineered in the step (b) may or may not be included in the present invention}. In an embodiment, in the step (b), the selection of the cell is performed on the basis of the expression of a plurality of distinguishable marker genes for positive selections respectively introduced in the alleles. In an embodiment, the step (b) is not performed by a method of estimating the number of engineered alleles on the basis of the expression strength of a single selective marker gene (e.g., the expression strength or fluorescence intensity of a fluorescent protein). This is because, in the case of selecting a cell by a method of estimating the number of engineered alleles on the basis of the expression strength of a single selective marker gene, a gene expression level varies among cells so that the cell in which the two or more alleles are engineered is difficult to completely separate from cells in which one allele is engineered; thus the step (b) requires single-cell cloning.

In the step (b), the selection of the cell can be appropriately performed according to the types of the selective marker genes used in the step (a). In this respect, the cell is selected on the basis of the expression of all the selective marker genes used in the step (a).

When the selective marker genes are, for example, positive selective marker genes, a cell expressing all the selective marker genes to be integrated (or integrated) in the chromosomal genome to be engineered can be selected. For example, a cell expressing the same number of positive selective markers as the number of the alleles to be engineered can be selected. When the positive selective marker genes are drug resistance genes, a cell expressing the positive selective markers can be selected by cell culture in a medium containing the drug(s). When the positive selective marker genes are fluorescent protein genes, luminescent enzyme genes, or chromogenic enzyme genes, a cell expressing the positive selective markers can be selected by selecting a cell emitting fluorescences, luminescences, or colors ascribable to the fluorescent proteins, the luminescent enzymes, or the chromogenic enzymes. In this step, when the same number of donor DNAs for selective markers as the number of the alleles to be engineered is incorporated in the genome, this number of the alleles is engineered. In a n-ploid cell, the number of alleles to be engineered is n or less. When the donor DNAs for selective markers, the number of types of which is equal to or more than this number and equal to or less than n, are incorporated in the genome, at least the alleles to be engineered (which are the two or more alleles) are engineered. In an embodiment, the number of the alleles to be engineered is n, and this number of types of the donor DNAs for selective markers is incorporated in the chromosomal genome; thus, all the alleles are engineered. In an embodiment, since the donor DNAs for selective markers, the number of types of which is equal to or more than the number of the alleles to be engineered, are used in this step, the number of positive selective markers expressed by the cell means that this number of alleles is reliably engineered. In the step (b), the number of the alleles to be engineered is preferably equal to the number of types of the donor DNAs for selective markers from the viewpoint of enhancing the selection efficiency of the cell.

As described above, in the genome engineering method of the present embodiment, HDR can be induced using n types of donor DNAs for selective markers for engineering n alleles in a n-ploid cell, to efficiently obtain a cell in which all the alleles carried by the cell are engineered. Since such a cell in which all the alleles are engineered can be reliably obtained, a cell having an engineered target region can be efficiently obtained even if the target region has a large size (e.g., 10 kbp or more). Hence, large-scale genome engineering is also achieved.

In an embodiment, in the step (b), an engineered cell can be selected, without cloning cells, from a pool containing cells obtained by the step (a). The omission of the cloning step can shorten a time required for the process. In an embodiment, the pool may contain 10⁵ or more, 10⁶ or more, 10⁷ or more, or 10⁸ or more cells.

### (Optional steps)

The genome engineering method of the present embodiment may comprise optional steps in addition to the step (a) and the step (b). Examples of the optional steps include the following steps (c) and (d):
(c) after the step (b), introducing, to the cell, a donor DNA for recombination comprising a desired nucleotide sequence between an upstream homology arm having a nucleotide sequence homologous to an upstream nucleotide sequence adjacent to the target region and a downstream homology arm having a nucleotide sequence homologous to a downstream nucleotide sequence adjacent to the target region; and
(d) after the step (c), selecting a cell not expressing the negative selective marker.

In an embodiment, the genome engineering method of the present embodiment may comprise optional steps in addition to the step (a) and the step (b). In an embodiment, in the genome engineering method or the method for obtaining a cell having an engineered genome according to the present embodiment, each of the two or more donor DNAs for selective markers has a selective marker gene for positive selection, a marker gene for negative selection other therethan, and a target sequence between the upstream homology arm and the downstream homology arm, wherein in the case of using the selective marker gene both for positive selection and for negative selection, the selective marker gene for negative selection is optionally absent, and the method may further comprise the following steps (c) and (d):
(c) after the step (b), introducing the following (iii) and (iv) to the selected cell to introduce a donor DNA for recombination to each of the two or more alleles:
   (iii) a genome engineering system comprising a sequence-specific nucleic acid cleaving molecule capable of targeting the additional target sequence and cleaving the additional target sequence, or a polynucleotide encoding the sequence-specific nucleic acid cleaving molecule, and
   (iv) a donor DNA for recombination comprising a desired nucleotide sequence, the donor DNA for recombination having an upstream homology arm having a nucleotide sequence that can be homologously recombined with an upstream nucleotide sequence of the target region and a downstream homology arm having a nucleotide sequence that can be homologously recombined with a downstream nucleotide sequence of the target region {the donor DNA for recombination may comprise a desired nucleotide sequence between the upstream homology arm and the downstream homology arm or may not comprise any nucleotide sequence therebetween}; and
(d) after the step (c), selecting a cell not expressing the marker gene for negative selection (step for negative selection).

### <Step (c)>

After the step (b), the step (c) may be performed. In an embodiment, in the step (c), a donor DNA for recombination comprising or not comprising a desired nucleotide sequence between an upstream homology arm and a downstream homology arm is introduced to the cell selected in the step (b). In an embodiment, in the step (c), a donor DNA for recombination comprising a desired nucleotide sequence between an upstream homology arm having a nucleotide sequence homologous to an upstream nucleotide sequence adjacent to the target region and a downstream homology arm having a nucleotide sequence homologous to a downstream nucleotide sequence adjacent to the target region is introduced to the cell selected in the step (b).

### <<Donor DNA for recombination>>

The donor DNA for recombination may comprise a desired nucleotide sequence to be knocked in. The desired nucleotide sequence is not particularly limited. For example, in the case of performing genome engineering for the purpose of knocking out the function of a gene contained in the target region, a nucleotide sequence lacking a portion or the whole of the nucleotide sequence of the target region can be used as the desired nucleotide sequence. In the case of integrating a foreign gene into the target region, a nucleotide sequence including the gene can be used as the desired nucleotide sequence. The size of the desired nucleotide sequence is not particularly limited and can be an arbitrary size. The desired nucleotide sequence can be, for example, 10 bp or more, 20 bp or more, 40 bp or more, 80 bp or more, 200 bp or more, 400 bp or more, 800 bp or more, 1 kbp or more, 2 kbp or more, 3 kbp or more, 4 kbp or more, 5 kbp or more, 6 kbp or more, 7 kbp or more, 8 kbp or more, 9 kbp or more, 10 kbp or more, 15 kbp or more, 20 kbp or more, 40 kbp or more, 80 kbp or more, 100 kbp or more, or 200 kbp or more. In the method of the present embodiment, a cell in which the desired nucleotide sequence is knocked in, in the two or more alleles can be efficiently selected. Hence, for example, DNA having a size as large as 5 kbp or more, 8 kbp or more, or 10 kbp or more can be knocked in. The donor DNA for recombination may be shorter than, for example, the donor DNAs for selective markers in terms of length.

The upstream homology arm and the downstream homology arm of the donor DNA for recombination may be the same as or different from those of the donor DNAs for selective markers. For the sake of convenience, the upstream homology arm and the downstream homology arm contained in each of the donor DNAs for selective markers are also referred to as a "first upstream homology arm" and a "first downstream homology arm", and the upstream homology arm and the downstream homology arm contained in the donor DNA for recombination are also referred to as a "second upstream homology arm" and a "second downstream homology arm". The second upstream homology arm and the second downstream homology arm are not particularly limited by their lengths and sequences as long as these homology arms can be homologously recombined with the first upstream homology arm or a region upstream therefrom and can be homologously recombined with the first downstream homology arm or a region downstream therefrom, for example (in an embodiment, the second upstream and downstream homology arms are not particularly limited by their lengths and sequences as long as these homology arms can be homologously recombined with neighboring regions of the target region). After recombination with the donor DNA for recombination, it is accepted that the nucleotide sequences of the donor DNAs for selective markers partially remain in the genome. Preferably, the nucleotide sequences of the donor DNAs for selective markers are completely removed from the genome by recombination with the donor DNA for recombination. Various genes loaded in the donor DNAs for selective markers are removed by recombination with the donor DNA for recombination. As a result, in an embodiment, each of the two or more alleles in the cell can be replaced with the donor DNA for recombination. In an embodiment, the donor DNA for recombination may have a desired nucleotide sequence. As a result, the cell in which the two or more alleles are engineered has the desired nucleotide sequence in the engineered alleles.

In the donor DNA for recombination, the desired nucleotide sequence is positioned between the second upstream homology arm and the second downstream homology arm. When the donor DNA for recombination comprises a foreign gene, it is preferred that the foreign gene should be operably linked to a promoter. The donor DNA for recombination may have, for example, an arbitrary control sequence such as an enhancer, a poly-A addition signal, or a terminator. When the donor DNA for recombination comprises a foreign gene, the donor DNA for recombination may have insulator sequences upstream and downstream of the foreign gene. In an embodiment, the donor DNA for recombination comprises a spacer sequence between the second upstream homology arm and the second downstream homology arm. In an embodiment, the donor DNA for recombination does not permit selection of a cell that has undergone homologous recombination with the donor DNA for recombination, if at the time of removal of a cell having the negative selective marker gene carried by the donor DNAs for selective markers, a gene that is the same as (or cannot be distinguished from) the negative selective marker gene is expressed under conditions in which its toxicity is exerted. Thus, the donor DNA for recombination is configured such that at the time of removal of a cell having the negative selective marker gene carried by the donor DNAs for selective markers, a gene that is the same as (or cannot be distinguished from) the negative selective marker gene is not expressed under conditions in which its toxicity is exerted. For example, in an embodiment, the donor DNA for recombination has neither a negative selective marker gene nor a second target sequence between the second upstream homology arm and the second downstream homology arm.

The donor DNA for recombination is preferably introduced, together with (i) described above, to the cell. As a result of introducing the donor DNA for recombination, together with (i) described above, to the cell, DNA of the target region is cleaved by the sequence-specific nucleic acid cleaving molecule of (i), followed by the knock-in of the desired nucleotide sequence of the donor DNA for recombination in the target region by HDR. Since the cell to which the donor DNA for recombination is to be introduced in this step is the cell selected in the step (b), each of the nucleotide sequences of the donor DNAs for selective markers is knocked in, in the target region. Hence, a target sequence of the genome engineering system (i) is a nucleotide sequence contained in the target region after knock-in of the donor DNAs for selective markers. For the sake of convenience, the target sequence of the genome engineering system in the step (a) is also referred to as a "first target sequence", and the target sequence of the genome engineering system in the step (c) is also referred to as a "second target sequence". An arbitrary sequence contained in the target region in the cell after the step (b) can be used as the second target sequence. In an embodiment, the second target sequence in each of the donor DNAs for selective markers can be a sequence that is absent in the genome of the cell. In an embodiment, the second target sequence in each of the donor DNAs for selective markers is a sequence that is absent in the genome of the cell and is a sequence different from the other sequences in the genome to an extent that the other sequences are not cleaved through off-target. In an embodiment, the second target sequence in each of the donor DNAs for selective markers can be a cleavage site of meganuclease that is absent in the genome. In an embodiment, the second target sequence is a region other than the negative selective marker gene in the step (d). As a matter of course, the donor DNA for recombination is configured such that homologous recombination with the donor DNA for recombination is not markedly inhibited. When the first target sequence remains in the target region in the cell after the step (b) or when the first target sequence is reintroduced by the donor DNAs for selective markers, the second target sequence may be the same as or different from the first target sequence.

The donor DNA for recombination may not comprise a nucleotide sequence between the upstream homology arm and the downstream homology arm and may comprise a nucleotide sequence of 10 bp or less, 20 bp or less, 30 bp or less, 40 bp or less, 50 bp or less, 60 bp or less, 70 bp or less, 80 bp or less, 90 bp or less, 100 bp or less, 200 bp or less, 300 bp or less, 400 bp or less, 500 bp or less, 600 bp or less, 700 bp or less, 800 bp or less, 900 bp or less, or 1 kbp or less between the upstream homology arm and the downstream homology arm. The donor DNA for recombination may comprise a nucleotide sequence of 1 kbp or more, 2 kbp or more, 3 kbp or more, 4 kbp or more, 5 kbp or more, 6 kbp or more, 7 kbp or more, 8 kbp or more, 9 kbp or more, or 10 kbp or more between the upstream homology arm and the downstream homology arm.

The donor DNA for recombination comprise one or more or all selected from the group consisting of a selective marker gene, a target sequence of site-specific recombinase, a gene encoding a factor having physiological activity, a gene encoding a factor having cytotoxicity, and a promoter sequence between the upstream homology arm and the downstream homology arm, or does not comprise one or more or all selected from this group therebetween.

In the step (c), the donor DNA for recombination is introduced to the cell selected in the step (b). In the cell selected in the step (b), each of the selective marker genes is knocked in, in the target region. The step (c) can be regarded as the step of removing the knocked-in selective marker gene in the target region or replacing the knocked-in selective marker gene with a desired nucleotide sequence.

### (Step (d))

After the step (c), the step (d) may be performed. In the step (d), a cell not expressing the negative selective marker is selected.

In the case of performing the step (d), donor DNAs for selective markers each comprising a positive selective marker gene and a negative selective marker gene can be used in the step (a). Specifically, each of the donor DNAs for selective markers for use in the step (a) can comprise a positive selective marker gene and a negative selective marker gene between the upstream homology arm and the downstream homology arm. The positional relationship between the positive selective marker gene and the negative selective marker gene is not particularly limited, and the positive selective marker gene may be located upstream of the negative selective marker gene, or vice versa. When each of the donor DNAs for selective markers has a positive selective marker gene and a negative selective marker gene, for example, a nucleotide sequence encoding a self-cleaving peptide, or an IRES (internal ribozyme entry site) sequence may intervene between the positive selective marker gene and the negative selective marker gene. The intervention of such a sequence allows the positive selective marker gene and the negative selective marker gene to be independently expressed from one promoter. Examples of 2A peptide include foot-and-mouth disease virus (FMDV)-derived 2A peptide (F2A), equine rhinitis A virus (ERAV)-derived 2A peptide (E2A), porcine teschovirus (PTV-1)-derived 2A peptide (P2A) and *Thosea asigna* virus (TaV)-derived 2A peptide (T2A).

Alternatively, the same selective marker gene may be used as a positive selective marker in the step (a) and used as a negative selective marker in the step (d). For example, when the selective marker genes are marker genes involved in color development (e.g., fluorescence or dyes) (visible marker genes) such as fluorescent protein genes, luminescent enzyme genes, or chromogenic enzyme genes, a cell emitting fluorescences, luminescences, or colors ascribable to the expression of the fluorescent proteins, the luminescent enzymes, or the chromogenic enzymes may be selected in the step (a) and a cell in which these fluorescences, luminescences, or colors have disappeared can be selected in the step (c). The case where the same selective marker gene serves both as a positive selective marker and as a negative selective marker is also encompassed by the case where each of the donor DNAs for selective markers has a positive selective marker as well as a negative selective marker.

The negative selective marker gene may be different or the same between or among the types of the donor DNAs for selective markers. Use of a common negative selective marker gene simplifies a cell selection operation in the step (d).

In the step (d), the selection of the cell can be appropriately performed according to the type of the negative selective marker gene used in the step (a). In this respect, a cell expressing none of the negative selective marker gene(s) used in the step (a) is selected.

For example, when the negative selective marker gene is a visible marker gene such as a fluorescent protein gene, a luminescent enzyme gene, or a chromogenic enzyme gene, a cell in which the visible marker such as fluorescence, luminescence or color has disappeared can be selected. When the negative selective marker gene is a suicide gene, a cell not expressing the negative selective marker can be selected by cell culture in a medium containing a drug that exerts toxicity by the expression of the suicide gene. In the case of using, for example, thymidine kinase gene, as the suicide gene, the cell can be cultured in a medium containing ganciclovir. The disappearance of the expression of the negative selective marker gene means that the negative selective marker gene integrated into the target region in the step (a) is replaced with the polynucleotide comprising the desired nucleotide sequence of the donor DNA for recombination. In this respect, the replacement with the polynucleotide is considered to occur for the whole nucleotide sequences knocked in, in the step (a). Hence, a cell in which each of the nucleotide sequences knocked in, in the step (a) is replaced with the desired nucleotide sequence of the donor DNA for recombination can be efficiently selected by selecting a cell in which the expression of the negative selective marker gene has disappeared. The negative selective marker gene such as a suicide gene may be operably linked to an inducible promoter. A cell not expressing the negative selective marker can be selected by cell culture in the presence of a drug that drives the inducible promoter such that the negative selective marker gene is expressed under conditions in which its toxicity is exerted. In this case, the negative selective marker gene may be a gene encoding a cytotoxin (e.g., ricin and diphtheria toxin) which causes toxicity to cells by only its expression.

In an embodiment, in the step (d), the cell in which the two or more alleles are engineered (cell in which the negative selective marker gene is absent) can be selected, without cloning cells, from a pool containing cells obtained by the step (c). In an embodiment, the pool may contain 10⁵ or more, 10⁶ or more, 10⁷ or more, or 10⁸ or more cells.

As described above, the step (c) and the step (d) can be performed to efficiently obtain a cell in which all the alleles carried by the cell are engineered into a desired sequence. Since such a cell in which all the alleles are engineered can be reliably obtained, a cell having a knocked-in desired nucleotide sequence in the target region can be efficiently obtained even if the desired nucleotide sequence has a large size (e.g., 10 kbp or more). In an embodiment, the step (c) and the step (d) are performed so that the target region is deleted in all the alleles carried by the cell and sequences upstream and downstream thereof (i.e., sequences that undergo homologous recombination with the upstream homology arm and the downstream homology arm, respectively) are seamlessly linked without one or more selected from the group consisting of base insertion, substitution, and deletion (e.g., without base insertion, substitution and deletion). In an embodiment, in the resulting cell, the upstream and downstream nucleotide sequences flanking the deleted region are seamlessly linked.

The number of live cells may be small or no live cell may be obtained by the step (b). This indicates that a gene that influences cell proliferation or survival is contained in the target region removed from the genome by homologous recombination with the upstream homology arm and the downstream homology arm. Thus, whether or not a gene that influences cell proliferation or survival is contained in the target region can be examined. In this case, the gene that influences cell proliferation or survival can be identified by changing the design positions of the upstream homology arm and the downstream homology arm and thereby changing a gene to be eliminated from the genome by homologous recombination. Thus, in the present invention, a step (e) can be performed after the step (b). Specifically, the step (e) comprises, when the number of live cells is small or no live cell is obtained by the step (b), identifying a gene that influences cell proliferation or survival by narrowing the target region and decreasing the number of genes to be eliminated from the genome. Provided that the target region contains only one gene, this gene is found to be the gene that influences cell proliferation or survival. After identification of the gene that influences cell proliferation or survival, a step (f) can be performed. The step (f) comprises knocking in the identified gene that influences cell proliferation or survival to another region (e.g., a safe harbor region) of the genome to be engineered {a donor DNA for recombination may be used in the knock-in}. As a result, a region to be deleted by the method of the present invention can be expanded (the target region can be extended upstream and/or downstream). The number of live cells that is small can be confirmed by comparison with the number of cells obtained by carrying out the steps (a) and (b) for a region that does not influence cell survival or proliferation. In an embodiment, a region that eliminates cell proliferation or survival may not be the target region in the step (a).

### [Genome engineering kit]

In one embodiment, the present invention provides a genome engineering kit for engineering two or more alleles in the chromosomal genome. The genome engineering kit comprises the following (i) and (ii):
(i) a genome engineering system comprising a sequence-specific nucleic acid cleaving molecule targeting a target region in the chromosomal genome, or a polynucleotide encoding the sequence-specific nucleic acid cleaving molecule, and
(ii) two or more donor DNAs for selective markers, each of which comprises a nucleotide sequence of a selective marker gene between a downstream homology arm having a nucleotide sequence homologous to an upstream nucleotide sequence adjacent to the target region and a downstream homology arm having a nucleotide sequence homologous to a downstream nucleotide sequence adjacent to the target region, the two or more donor DNAs for selective markers respectively having different selective marker genes, wherein the number of types of the donor DNAs for selective markers is equal to or more than the number of the alleles that are subject to genome engineering.

In one embodiment, the present invention provides a genome engineering kit for engineering two or more alleles in the chromosomal genome, comprising the following (i) and (ii):
(i) a genome engineering system comprising a sequence-specific nucleic acid cleaving molecule capable of targeting a target region in the chromosomal genome and cleaving the target region, or a polynucleotide encoding the sequence-specific nucleic acid cleaving molecule, and
(ii) two or more donor DNAs for selective markers, each of which has an upstream homology arm having a nucleotide sequence that can be homologously recombined with an upstream nucleotide sequence of the target region and a downstream homology arm having a nucleotide sequence that can be homologously recombined with a downstream nucleotide sequence of the target region, and comprises a nucleotide sequence of a selective marker gene between the upstream homology arm and the downstream homology arm, the two or more donor DNAs for selective markers respectively having selective marker genes distinguishable from each other, wherein the number of types of the donor DNAs for selective markers is equal to or more than the number of the alleles that are subject to genome engineering.

In this embodiment, the selective marker gene may be unique to each type of donor DNA for the selective marker. The kit may be used in the method of the present invention. The kit may be used in the method for preparing a cell in which two or more alleles in the chromosomal genome are engineered.

(i) and (ii) contained in the kit of the present embodiment are the same as (i) and (ii) described in the section [Genome engineering method]. Use of the kit of the present embodiment enables the genome engineering method to be easily performed.

In one embodiment, the present invention provides a cell in which two or more alleles in the chromosomal genome are engineered, the cell having different (distinguishable) selective marker genes in the two or more alleles, respectively. In an embodiment, the cell can be a cell of a single-celled organism. In an embodiment, the cell can be an isolated cell. In an embodiment, the cell can be a cell selected from the group consisting of pluripotent or multipotent cells and pluripotent stem cells (embryonic stem cells and induced pluripotent stem cells, etc.). In an embodiment, the cell can be a tissue stem cell. In an embodiment, the cell can be a somatic cell. In an embodiment, the cell can be a germ-line cell (e.g., a germ cell). In an embodiment, the cell can be a cell line. In an embodiment, the cell can be an immortalized cell. In an embodiment, the cell can be a cancer cell. In an embodiment, the cell can be a non-cancer cell. In an embodiment, the cell can be a cell of a patient with a disease. In an embodiment, the cell can be a cell of a healthy individual. In an embodiment, the cell can be a cell selected from the group consisting of animal cells (e.g., human cells), for example, insect cells (e.g., silkworm cells), HEK293 cells, HEK293T cells, Expi293F(TM) cells, FreeStyle(TM) 293F cells, Chinese hamster ovary cells (CHO cells), CHO-S cells, CHO-K1 cells, and ExpiCHO cells, and cells derived from these cells. In a preferred embodiment, in the cell, all the alleles in target regions in the chromosomal genome are engineered, and the regions thus engineered respectively have different (distinguishable) selective marker genes.

In one embodiment, the present invention provides a method for culturing a cell in which two or more alleles in the chromosomal genome are engineered, the cell having different (distinguishable) selective marker genes in the two or more alleles, respectively. When the selective marker genes are drug resistance marker genes, the culture can be culture in the presence of respective drugs for the drug resistance marker genes. The culture can be performed under conditions suitable for the maintenance or proliferation of the cell.

In one embodiment, the present invention provides a non-human organism having the chromosomal genome in which two or more alleles are engineered, the non-human organism having different (distinguishable) selective marker genes in the two or more alleles, respectively. In an embodiment, the cell can be a cell of a single-celled organism. In an embodiment, the non-human organism can be an organism selected from yeasts (e.g., fission yeasts and budding yeasts, for example, yeasts of the genus *Saccharomyces* such as *Saccharomyces cerevisiae, Saccharomyces carlsbergensis, Saccharomyces fragilis,* and *Saccharomyces rouxii,* the genus *Candida* such as *Candida utilis* and *Candida tropicalis,* the genus *Pichia,* the genus *Kluyveromyces,* the genus *Yarrowia,* the genus *Hansenula,* and the genus *Endomyces.* In an embodiment, the non-human organism can be a filamentous bacterium (e.g., *Aspergillus, Trichoderma, Humicola, Acremonium, Fusarium,* and *Penicillium* species). In an embodiment, the non-human organism can be a multi-celled organism. In an embodiment, the non-human organism can be a non-human animal. In an embodiment, the non-human organism can be a plant. In a preferred embodiment, in the non-human organism, all the alleles in target regions in the chromosomal genome are engineered, and the regions thus engineered respectively have different (distinguishable) selective marker genes.

In the cell, one or more genes necessary for cell survival or proliferation may be contained or gathered in another region of the chromosomal genome. Another region can be, for example, a safe harbor region (e.g., an AAVS1 region).

### [Examples]

Hereinafter, the present invention will be described with reference to Experimental Examples. However, the present invention is not limited by Examples given below.

### [Experimental Example 1]

### Preparation of donor DNAs

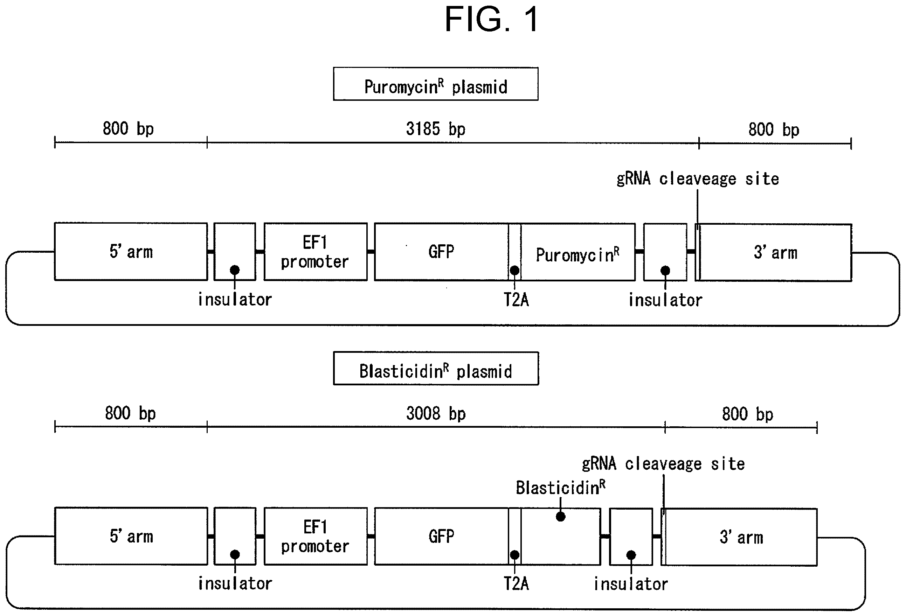

In order to screen for a cell in which two alleles were edited, two types of donor DNAs differing in positive selective markers were prepared (Figure 1; Puromycin(R) plasmid and Blasticidin(R) plasmid). The negative selective marker used was GFP gene. In the Puromycin(R) plasmid, the GFP gene is linked downstream of EF1 promoter, and puromycin resistance gene is further linked so as to flank a T2A sequence. In the Blasticidin(R) plasmid, the GFP gene is linked downstream of EF1 promoter, and blasticidin resistance gene is further linked so as to flank a T2A sequence.

HR110PA-1 plasmid (Funakoshi Co., Ltd.) was used as a backbone sequence for the donor plasmids (donor DNAs). This HR110PA-1 plasmid was cleaved with restriction enzymes EcoRI and BamHI to excise a sequence having a replication origin and ampicillin resistance gene, followed by purification. The resultant was then used as the backbone sequence for all the donor plasmids.

The selective marker sequences and homology arm sequences of the plasmids were amplified by the following PCR. The EF1 promoter sequence was prepared by amplification from an EcoRI recognition sequence to the EF1 promoter sequence on HR110PA-1. The GFP sequence was prepared with CS-CDF-CG-PRE plasmid (dnaconda.riken.jp/search/RDB_clone/RDB04/RDB04379.html) as a template. The T2A sequence to the puromycin expression sequence was prepared by amplification from the T2A sequence to a BamHI recognition sequence on HR110PA-1. The upstream homology arm (810 bp: SEQ ID NO: 2) and the downstream homology arm (792 bp: SEQ ID NO: 3) were prepared with the genome of HCT116 as a template.

A 20 bp homologous sequence corresponding to a flanking sequence was added, at the time of PCR, to each of the selective marker sequences and the homology arm sequences. The backbone sequence was attached to the selective marker sequences and the homology arm sequences via this homologous sequence using NEBuilder HiFi DNA Assembly (NEW ENGLAND BioLabs) and introduced into E. *coli,* which was then cultured in an ampicillinsupplemented medium for the cloning of the puromycin plasmid.

The blasticidin plasmid was prepared by engineering the puromycin plasmid. A blasticidin ORF sequence amplified by PCR and a sequence amplified, except for the puromycin ORF sequence, with the puromycin plasmid as a template were attached using NEBuilder HiFi DNA Assembly, and cloning was performed in the same manner as in the puromycin plasmid preparation.

### [Experimental Example 2]

Introduction of selective markers to first intron of TP53 gene

### (Cells and target region)

The cells used were colorectal cancer-derived cell line HCT116 cells. Most of cells of this cell line are diploids. The first intron of cancer suppressor gene TP53 was selected as a target region of genome editing.

### (Step (a))

The Puromycin(R) plasmid and the Blasticidin(R) plasmid (12.5 ng each) were introduced as the donor plasmids (donor DNAs) to 10⁵ HCT116 cells.

On the day before donor plasmid introduction, 10⁵ HCT116 cells per well were seeded to a 24-well plate. For culture, 500 µL of McCoy's 5A medium (adjusted to 10% by the addition of FBS) per well was used.

On the day following seeding of the cells, a plasmid introduction solution was prepared according to the following composition.

| | |
|---|---|
| Opti-MEM | 100 µL |
| Cas9&gRNA coexpression plasmid (pX330-U6-Chimeric_BB-CBh-hSpCas9; Addgene, plasmid No. 42230) | 475 ng |
| The Puromycin(R) plasmid | 12.5 ng |
| The Blasticidin(R) plasmid | 12.5 ng |
| FuGENE HD | 1.5 µL |

The target sequence of gRNA used in the Cas9&gRNA coexpression plasmid is given below.
CTCAGAGGGGGCTCGACGCTAGG (SEQ ID NO: 4)

The plasmid introduction solution was mixed by pipetting, then incubated at room temperature for 10 minutes, and then added to the 24-well plate.

### (Step (b))

After introduction of both the plasmids, the cells were cultured for 8 days in McCoy's 5A medium (adjusted to 10% by the addition of FBS) supplemented with 1 µg/mL puromycin and 10 µg/mL blasticidin. The cells thus cultured were recovered from independent colonies by pipetting.

### (Confirmation of selective marker gene knock-in)

DNAs were extracted from 27 recovered cell clones by the phenol/chloroform extraction method, and the knock-in of the selective marker genes was confirmed by junction PCR.

The HCT116 cells were recovered into a 1.5 mL tube and centrifuged at 150 g at room temperature for 3 minutes. The supernatant was removed, and the cells were resuspended in 173 µL of a TE buffer (100 mM NaCl). The cell suspension was incubated at 95°C for 5 minutes and then cooled to room temperature. 20 µL of a TE buffer (100 mM NaCl, 0.5% SDS), 5 µL of proteinase K (5 mg/ml), and 2 µL of RNase A (100 mg/ml) were added thereto and mixed, and the mixture was then inverted and stirred at 37°C for 2 hours. The resulting mixture was incubated at 85°C for 25 minutes. Then, 200 µL of phenol/chloroform/isoamyl alcohol (25:24:1) was added thereto, and the mixture was inverted and stirred for 10 minutes. The resulting mixture was centrifuged at 15,000 g at room temperature for 10 minutes, and the upper layer was then transferred to a fresh 1.5 mL tube. Chloroform in the same amount as that of the transferred upper layer was added to the 1.5 mL tube, and the mixture was inverted and stirred for 10 minutes. The resulting mixture was centrifuged at 15,000 g at room temperature for 10 minutes, and the upper layer was then transferred to a fresh 1.5 mL tube. Isopropanol in the same amount as that of the transferred upper layer was added to the 1.5 mL tube, and the mixture was centrifuged at 15,000 g at 4°C for 30 minutes. The supernatant was removed, and 150 µL of 70% ethanol was added to the residue, followed by centrifugation at 15,000 g at 4°C for 5 minutes. The supernatant was completely removed, and the precipitate was dissolved in 30 µL of a TE buffer.

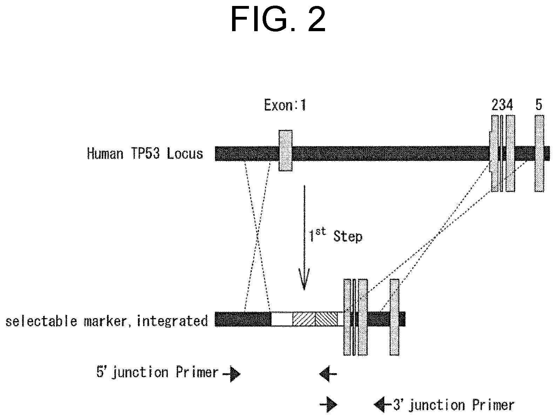

Junction PCR was performed with the DNAs thus recovered as templates using the junction primers shown in Figure 2. The junction primers are primers that amplify the puromycin resistance gene or the blasticidin resistance gene so as to span both the 5' and 3' junctions. The PCR products were subjected to agarose gel electrophoresis to confirm amplified DNA fragments. The PCR conditions and the primer sequences are given below.

### <PCR conditions>

Composition of PCR solution (reaction system: 25 µL)
1 × KOD one (Toyobo Co., Ltd.)
0.2 µM forward primer
0.2 µM reverse primer
10 ng of genomic DNA

### Thermal cycler conditions

98°C for 30 sec
98°C for 10 sec; 68°C for 20 sec, 50 cycles 4°C

### <Primer sequences>

PCR primers for Puromycin 5' junction
Forward primer: TGCCCCGTTGTTATCCTTAC (SEQ ID NO: 5)
Reverse primer: GCTCGTAGAAGGGGAGGTTG (SEQ ID NO: 6)

PCR primers for Puromycin 3' junction
Forward primer: GTCACCGAGCTGCAAGAAC (SEQ ID NO: 7)
Reverse primer: GAAGACGGCAGCAAAGAAAC (SEQ ID NO: 8)

PCR primers for Blasticidin 5' junction
Forward primer: TGCCCCGTTGTTATCCTTAC (SEQ ID NO: 9)
Reverse primer: GCTTCAATATGTACTGCCGAAA (SEQ ID NO: 10)

PCR primers for Blasticidin 3' junction
Forward primer: GAAGCCATTGCGATTGGTAG (SEQ ID NO: 11)
Reverse primer: GAAGACGGCAGCAAAGAAAC (SEQ ID NO: 12)

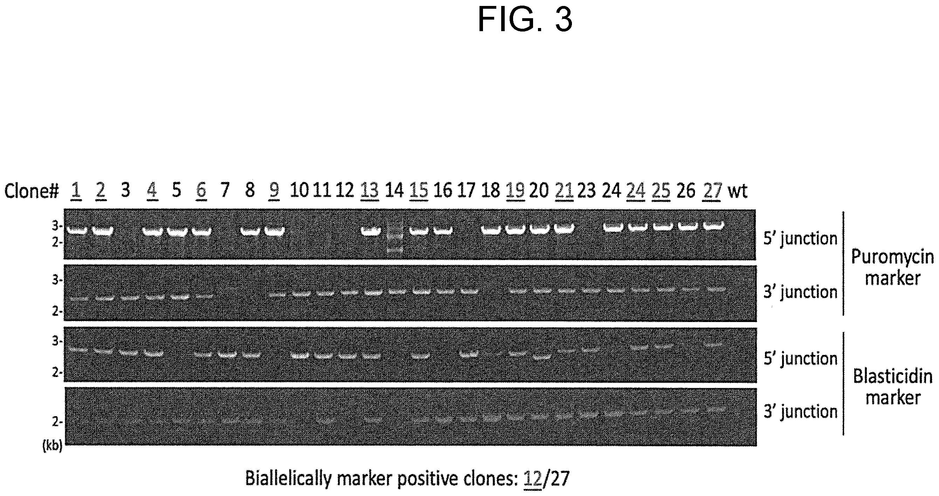

The results are shown in Figure 3. From the DNA extracted from wild-type HCT116, no band of expected size was detected both for the puromycin resistance gene and for the blasticidin resistance gene (rightmost lane: WT). On the other hand, from the DNAs extracted from the cell clones obtained by the step (a) and the step (b), all bands of expected size were detected both for the puromycin resistance gene and for the blasticidin resistance gene in 12 out of 27 clones (underlined clones). These results indicate that in these 12 clones, one of the alleles in the first intron of TP53 was replaced with the puromycin resistance gene and the other allele was replaced with the blasticidin resistance gene. As for clone 13, the nucleotide sequences of the PCR products were subjected to sequence analysis. As a result, both of a sequence with the puromycin resistance gene replaced for the first intron of TP53 and a sequence with the blasticidin resistance gene replaced for the first intron of TP53 were confirmed.

### [Reference Experimental Example]

The Puromycin(R) plasmid was introduced to HCT116 cells in the same manner as above except that the Blasticidin(R) plasmid was not used.

After introduction of the Puromycin(R) plasmid, the cells were cultured for 8 days in McCoy's 5A medium (adjusted to 10% by the addition of FBS) supplemented with 1 µg/mL puromycin. The cells thus cultured were recovered from independent colonies by pipetting.

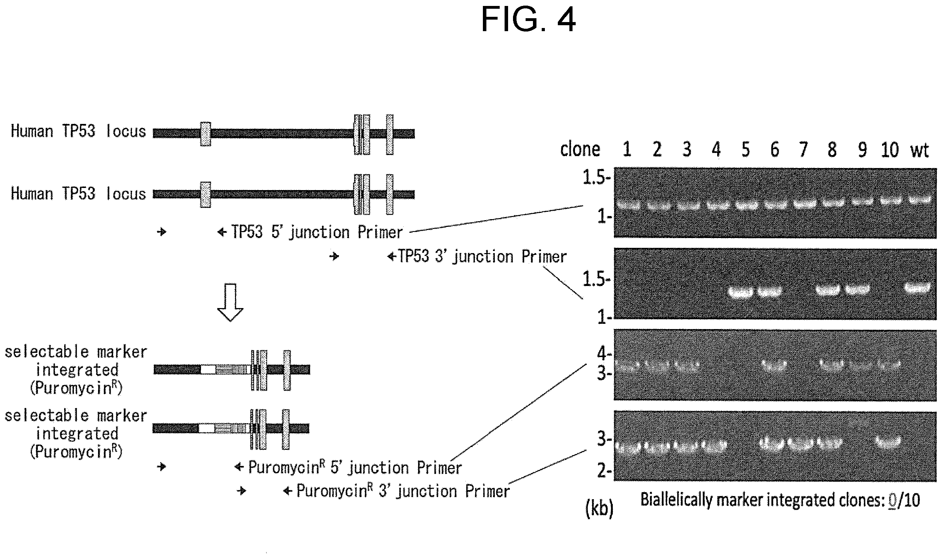

DNAs were extracted from 10 recovered cell clones in the same manner as above. Junction PCR was performed in the same manner as above using junction primers (see the left diagram of Figure 4) that amplified the puromycin resistance gene so as to span both 5' and 3' junctions. The PCR products were subjected to agarose gel electrophoresis to confirm amplified DNA fragments.

As a result, any clone in which both the alleles in the first intron of TP53 were replaced with the puromycin resistance gene was not obtained. Also, the obtainment efficiency of a clone in which only one of the alleles was replaced with the puromycin resistance gene was low (right diagram of Figure 4).

### [Experimental Example 3]

### Removal of selective markers

### (Step (c))

A plasmid containing a wild-type TP53 sequence (wild-type TP53 plasmid) was prepared as a donor DNA. The wild-type TP53 plasmid was introduced to the cell clone obtained in Experimental Example 2 (clone #13 in Figure 3) in the same manner as above except that the donor DNA used was a plasmid containing the sequences of the first intron of wild-type TP53 and its neighboring regions (5' arm and 3' arm). The target sequence of gRNA used in the Cas9&gRNA coexpression plasmid is given below.

### GGCGCAACGCGATCGCGTAAGGG (SEQ ID NO: 13)

### (Step (d))

After introduction of the wild-type TP53 plasmid, a cell clone in which GFP expression disappeared was screened for using a cell sorter (SH800, Sony Corp.) and recovered into a 96-well plate on a cell basis.

### (Confirmation of marker gene removal)

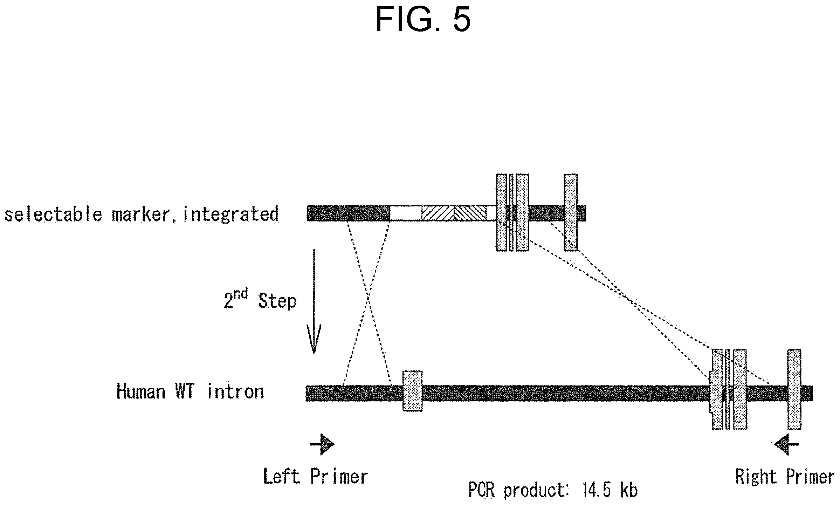

DNAs were extracted from the recovered cells in the same manner as above. PCR was performed using the primers shown in Figure 5. The PCR products were subjected to agarose gel electrophoresis to confirm amplified DNA fragments. The PCR solution has the same composition as above. The thermal cycler conditions and the primer sequences are given below.

### Thermal cycler conditions

98°C for 30 sec
98°C for 10 sec; 68°C for 2.5 min, 50 cycles 4°C

### <Primer sequences>

### PCR primers for TP53 first intron amplification

Forward primer: TGCCCCGTTGTTATCCTTAC (SEQ ID NO: 14)
Reverse primer: GAAGACGGCAGCAAAGAAAC (SEQ ID NO: 15)

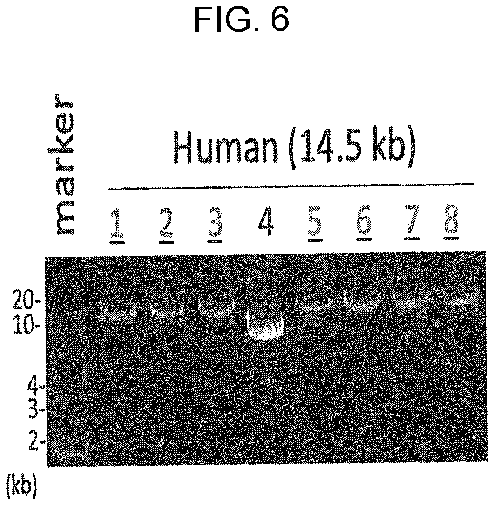

The results are shown in Figure 6. Bands of expected size were detected in 7 out of 8 clones (underlined clones). These results indicate that in these 7 clones, the selective markers (puromycin resistance gene or blasticidin resistance gene, and GFP gene) were replaced with the first intron sequence of the wild-type TP53 gene. A 5.6 kb band, which is detected when the selective markers remain in one of the alleles, was not detected in the 7 clones. These results suggest that in both the alleles, the selective markers were replaced with the wild-type first intron.

### [Experimental Example 4]

### Engineering of first intron of TP53 gene

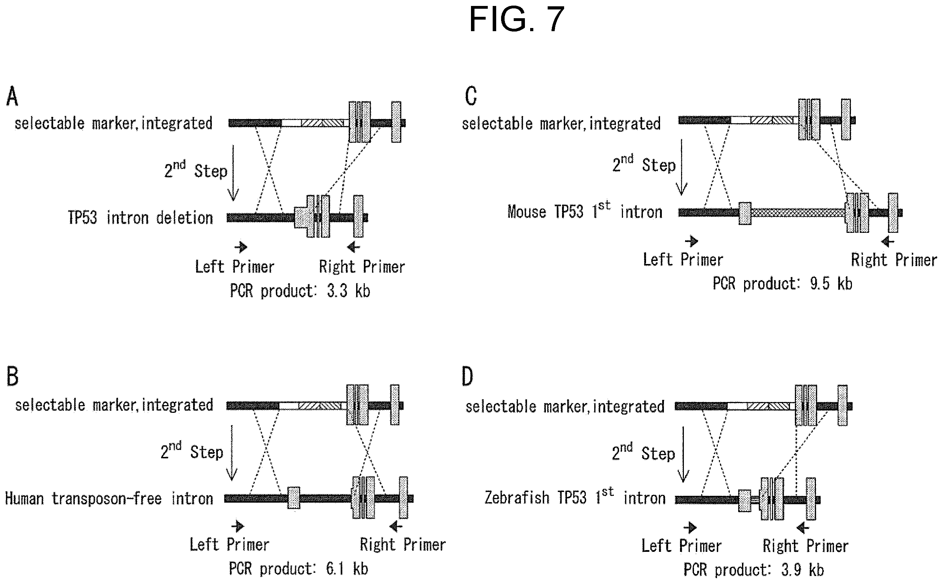

The step (c) and the step (d) were performed in the same manner as in Experimental Example 3 except that the donor DNAs used were the plasmids respectively shown in Figures 7A to 7D.

DNAs were extracted from the recovered cells in the same manner as above. PCR was performed using the primers respectively shown in Figures 7A to 7D. The PCR products were subjected to agarose gel electrophoresis to confirm amplified DNA fragments. The PCR solution has the same composition as above. The thermal cycler conditions and the primer sequences are given below.

### Thermal cycler conditions

98°C for 30 sec
98°C for 10 sec; 68°C for 2.5 min, 50 cycles 4°C

### <Primer sequences>

### PCR primers for TP53 first intron amplification

Forward primer: TGCCCCGTTGTTATCCTTAC (SEQ ID NO: 14)
Reverse primer: GAAGACGGCAGCAAAGAAAC (SEQ ID NO: 15)

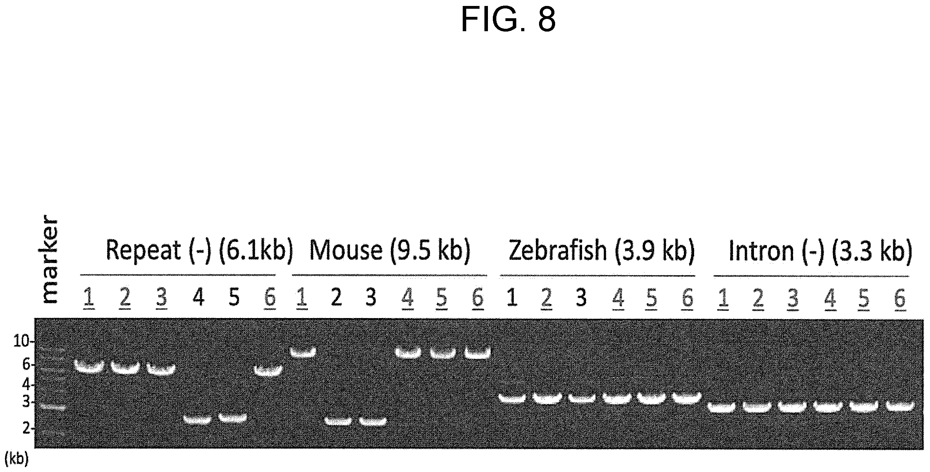

The results are shown in Figure 8. In the case of using any of the donor DNAs, cell clones in which both the alleles were replaced with the sequence in the donor DNA were obtained with high probability (underlined clones) .

The first intron of the human TP53 gene is a relatively large genomic region of 10,762 bp. The method described above was confirmed to be able to efficiently genome-edit both the alleles as well as to be able to efficiently perform genome editing even for such a relatively large genomic region.

### [Example 5]

In Example 4, the first intron of the human TP53 gene in HCT116 cells was deleted. In this Example, the deletion of the first intron of the human TP53 gene was attempted in human pluripotent stem cells instead of the HCT116 cells. Specifically, the human pluripotent stem cells were human induced pluripotent stem cells (iPS cells).

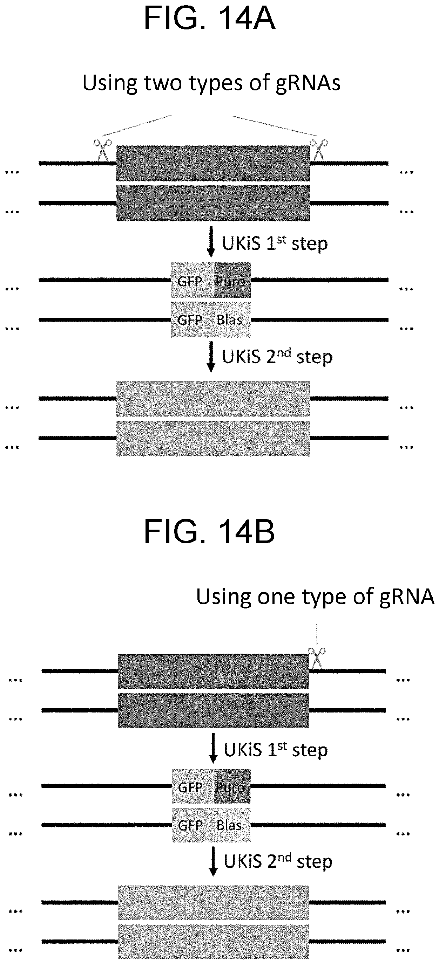

In this Example, cleavage was introduced using CRISPR/Cas9 system having gRNAs designed so as to be able to induce specific cleavage upstream and downstream of the target gene in the presence of donor DNAs for selective markers, as shown in Figure 14A. The cleavage locations were designed so as to reside between a region to be hybridized with the upstream homology arm and the target gene, and between a region to be hybridized with the downstream homology arm and the target gene. This induced the first stage of recombination. The sequences of the gRNAs used are as follows.

### <Guide RNAs>

Human TP53 upstream gRNA: CUCAGAGGGGGCUCGACGCU (SEQ ID NO: 16)
Human TP53 downstream gRNA: GGUGCUUUAAGAAUUACCGC (SEQ ID NO: 17)

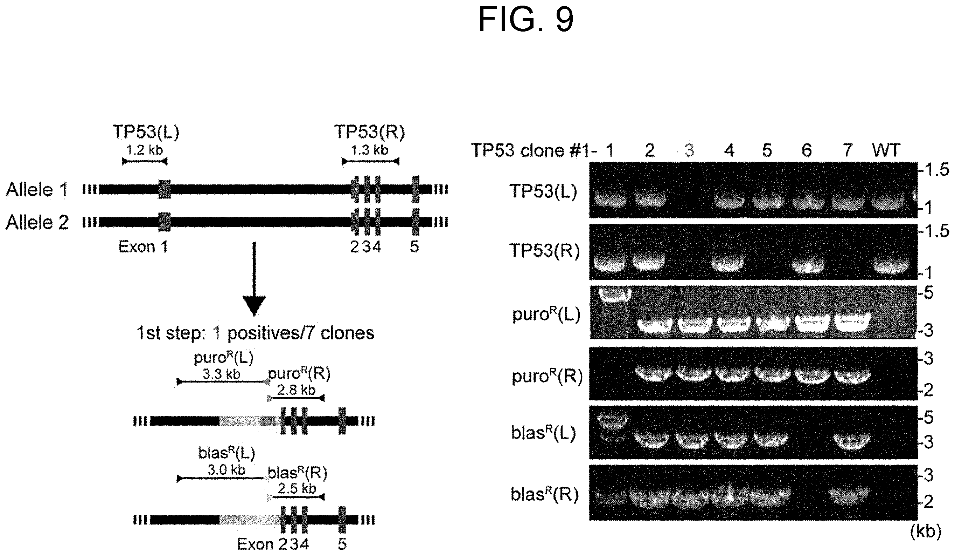

In the presence of the two types of donor DNAs for selective markers used in Example 4, cleavage was made upstream and downstream of the first intron of the human TP53 gene in the genome of the human iPS cells. Then, cells having the genome in which the puromycin resistance gene and the blasticidin resistance gene were respectively introduced in the alleles were obtained in the presence of puromycin and blasticidin, and cloned. The TP53 gene, the puromycin resistance gene and the blasticidin resistance gene were each amplified using the primers described above. The results were as shown in Figure 9. As shown in Figure 9, cells were obtained in which in both the alleles, the first intron was replaced with the donor DNA sequence (i.e., cells in which the first intron of the human TP53 gene was deleted in both the alleles).

### [Example 6]

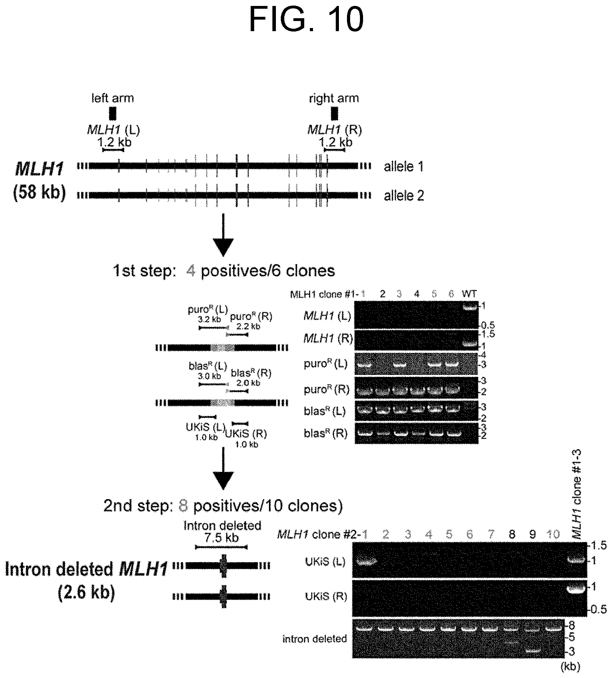

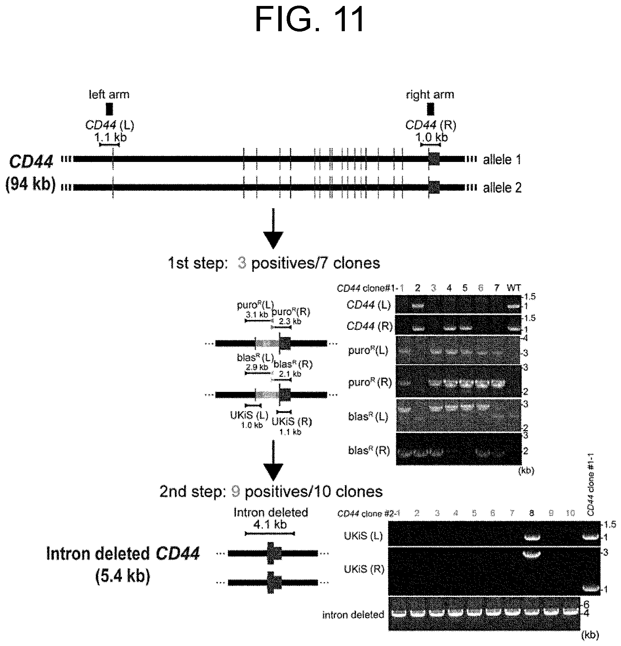

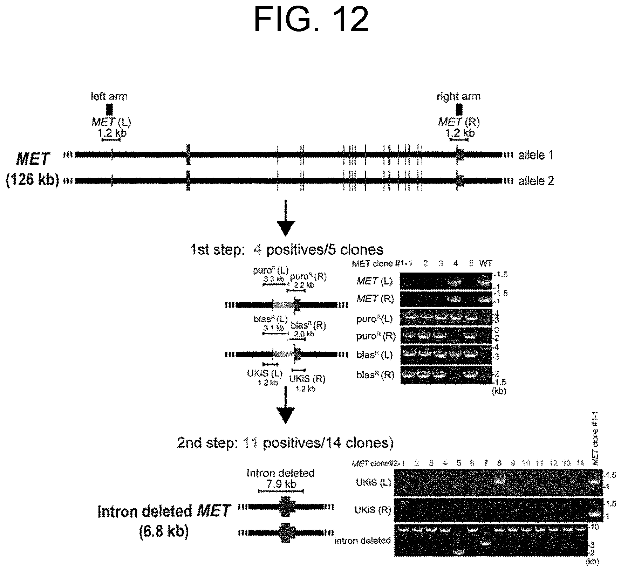

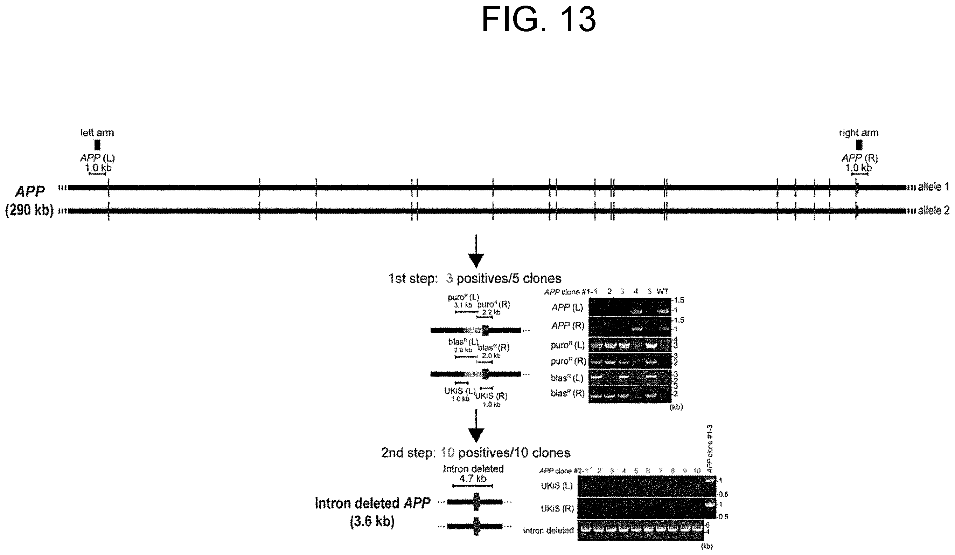

As in TP53, gene deletion was induced by the application of the method described above to each of human genes encoding MLH1, CD44, MET, and APP (hereinafter, referred to as target genes) in HCT116 cells. As respectively shown in Figures 10 to 13, the sizes of the target genes were 58 kb for MLH1, 94 kb for CD44, 126 kb for MET, and 290 kb for APP. An upstream homology arm and a downstream homology arm were respectively designed for the upstream and downstream regions of each of these target genes. Donor DNAs for selective markers were provided which were loaded with their respective distinguishably different marker genes for selections and a visible marker gene between the arms. Specifically, one of the donor DNAs for selective markers had GFP and the puromycin resistance gene, and the other donor DNA for the selective marker had GFP and the blasticidin resistance gene.

Cleavage was introduced using CRISPR/Cas9 system having gRNAs designed so as to be able to induce specific cleavage upstream and downstream of the target gene in the presence of donor DNAs for selective markers, as shown in Figure 14A. The cleavage locations were designed so as to reside between a region to be hybridized with the upstream homology arm and the target gene, and between a region to be hybridized with the downstream homology arm and the target gene. This induced the first stage of recombination.

### <Sequences of guide RNAs>

The sequences of the guide RNAs for the upstream and downstream regions of each target gene were as follows.
MLH1 upstream gRNA: GCGCCUGACGUCGCGUUCGC (SEQ ID NO: 18)
MLH1 downstream gRNA: GGAGGCCUUGGCACGGGUUC (SEQ ID NO: 19)
CD44 upstream gRNA: CGAGGAUGGCGGACCGAACC (SEQ ID NO: 20)
CD44 downstream gRNA: GCCAAGUGGACUCAACGGAG (SEQ ID NO: 21)
MET upstream gRNA: GGGCCGCGCGCGCCGAUGCC (SEQ ID NO: 22)
MET1 downstream gRNA: GUUCCCACCUCGCAAGCAAU (SEQ ID NO: 23)
APP upstream gRNA: CUCCCGGGGGUGUCGUAUAA (SEQ ID NO: 24)
APP downstream gRNA: UUCUAUAAAUGGACACCGAU (SEQ ID NO: 25)

### <Primer sequences>

The deletion of each target gene and the introduction of the drug resistance genes were detected using the following primers.
PCR primers for MLH1 amplification
PCR primers for MLH1 5' junction
   Forward primer: CCAAGAACGCTTCCATTTCT (SEQ ID NO: 26)
   Reverse primer: CCCTGTGCCTGGTCTGTC (SEQ ID NO: 27) PCR primers for MLH1 3' junction
   Forward primer: TTCTGAGGTCTCCAGCAAGT (SEQ ID NO: 28)
   Reverse primer: AAGTTGAAGATGAATTGAAAGCAG (SEQ ID NO: 29)
PCR primers for Puromycin 5' junction
   Forward primer: CCAAGAACGCTTCCATTTCT (SEQ ID NO: 30)
   Reverse primer: GCTCGTAGAAGGGGAGGTTG (SEQ ID NO: 31)
PCR primers for Puromycin 3' junction
   Forward primer: GTCACCGAGCTGCAAGAAC (SEQ ID NO: 32)
   Reverse primer: AAGTTGAAGATGAATTGAAAGCAG (SEQ ID NO: 33)
PCR primers for Blasticidin 5' junction
   Forward primer: CCAAGAACGCTTCCATTTCT (SEQ ID NO: 34)
   Reverse primer: GCTTCAATATGTACTGCCGAAA (SEQ ID NO: 35)
PCR primers for Blasticidin 3' junction
   Forward primer: GAAGCCATTGCGATTGGTAG (SEQ ID NO: 36)
   Reverse primer: AAGTTGAAGATGAATTGAAAGCAG (SEQ ID NO: 38)
PCR primers for CD44 amplification
PCR primers for CD44 5' junction
   Forward primer: AGTGGATGGACAGGAGGATG (SEQ ID NO: 39)
   Reverse primer: GCGAAAGGAGCTGGAGGA (SEQ ID NO: 40)
PCR primers for CD44 3' junction
   Forward primer: ATGGAGCTGTGGAGGACAGA (SEQ ID NO: 41)
   Reverse primer: GAGTGGGTCTGAGTGGGAAC (SEQ ID NO: 42)
PCR primers for Puromycin 5' junction
   Forward primer: AGTGGATGGACAGGAGGATG (SEQ ID NO: 43)
   Reverse primer: GCTCGTAGAAGGGGAGGTTG (SEQ ID NO: 44)
PCR primers for Puromycin 3' junction
   Forward primer: GTCACCGAGCTGCAAGAAC (SEQ ID NO: 45)
   Reverse primer: GAGTGGGTCTGAGTGGGAAC (SEQ ID NO: 46)
PCR primers for Blasticidin 5' junction
   Forward primer: AGTGGATGGACAGGAGGATG (SEQ ID NO: 47)
   Reverse primer: GCTTCAATATGTACTGCCGAAA (SEQ ID NO: 48)
PCR primers for Blasticidin 3' junction
   Forward primer: GAAGCCATTGCGATTGGTAG (SEQ ID NO: 49)
   Reverse primer: GAGTGGGTCTGAGTGGGAAC (SEQ ID NO: 50)
PCR primers for MET amplification
PCR primers for MET 5' junction
   Forward primer: TGAAATCACTCTTATGTAACCTCTGG (SEQ ID NO: 51)
   Reverse primer: AAGGGGCTGCAATTTTACCT (SEQ ID NO: 52) PCR primers for MET 3' junction
   Forward primer: GGGTGGATGGATTGAAAAGA (SEQ ID NO: 53)
   Reverse primer: TGCAGGTATAGGCAGTGACAAG (SEQ ID NO: 54)
PCR primers for Puromycin 5' junction
   Forward primer: TGAAATCACTCTTATGTAACCTCTGG (SEQ ID NO: 55)
   Reverse primer: GCTCGTAGAAGGGGAGGTTG (SEQ ID NO: 56) PCR primers for Puromycin 3' junction
   Forward primer: GTCACCGAGCTGCAAGAAC (SEQ ID NO: 57)
   Reverse primer: TGCAGGTATAGGCAGTGACAAG (SEQ ID NO: 58)
PCR primers for Blasticidin 5' junction
   Forward primer: TGAAATCACTCTTATGTAACCTCTGG (SEQ ID NO: 59)
   Reverse primer: GCTTCAATATGTACTGCCGAAA (SEQ ID NO: 60)
PCR primers for Blasticidin 3' junction
   Forward primer: GAAGCCATTGCGATTGGTAG (SEQ ID NO: 61)
   Reverse primer: TGCAGGTATAGGCAGTGACAAG (SEQ ID NO: 62)
PCR primers for APP amplification
PCR primers for APP 5' junction
   Forward primer: GGGGAGCTGGTACAGAAATG (SEQ ID NO: 63)
   Reverse primer: CAGGATCAGGGAAAGGTGAG (SEQ ID NO: 64) PCR primers for APP 3' junction
   Forward primer: GAACGGCTACGAAAATCCAA (SEQ ID NO: 65)
   Reverse primer: CTCTTCTCCCCACCCAAAA (SEQ ID NO: 66)
PCR primers for Puromycin 5' junction
   Forward primer: GGGGAGCTGGTACAGAAATG (SEQ ID NO: 67)
   Reverse primer: GCTCGTAGAAGGGGAGGTTG (SEQ ID NO: 68) PCR primers for Puromycin 3' junction
   Forward primer: GTCACCGAGCTGCAAGAAC (SEQ ID NO: 69)
   Reverse primer: CTCTTCTCCCCACCCAAAA (SEQ ID NO: 70)
PCR primers for Blasticidin 5' junction
   Forward primer: GGGGAGCTGGTACAGAAATG (SEQ ID NO: 71)
   Reverse primer: GCTTCAATATGTACTGCCGAAA (SEQ ID NO: 72)
PCR primers for Blasticidin 3' junction
   Forward primer: GAAGCCATTGCGATTGGTAG (SEQ ID NO: 73)
   Reverse primer: CTCTTCTCCCCACCCAAAA (SEQ ID NO: 74)

As a result, as shown in "1st step" in Figures 10 to 13, a plurality of clones in which the target gene was deleted in both the alleles were obtained for each target gene. The obtainment efficiency of the clones in which the target gene was deleted in both the alleles was higher when cleavage was introduced both upstream and downstream of the target gene in the presence of the donor DNAs for selective markers, as shown in Figure 14A, than when cleavage was induced only downstream or only upstream of the target gene in the presence of the donor DNAs for selective markers, as shown in Figure 14B. In this way, the target region in each of the genes described above was able to be replaced with the sequences of the donor DNAs for selective markers.

Next, the introduced selective markers were removed by the second stage of recombination, instead of deleting the target gene. Here, the visible marker GFP was used as an index. Cells lacking regions containing the selective markers were selected with no expression of GFP as an index. As a result, as shown in "2nd step" of Figures 10 to 13, the selective markers including GFP were able to be removed from both the alleles in the genome.

### [Industrial Applicability]

According to the present invention, two or more alleles can be efficiently engineered, and a genome engineering method and a genome engineering kit which are capable of engineering a relatively large region can be provided.

### [Sequence Listing]

[Final] PL19-0001_ST25.txt

## Claims

1. A method for preparing a cell in which two or more alleles in the chromosomal genome are engineered, comprising the steps of:
(a) introducing the following (i) and (ii) to a cell comprising two or more alleles to introduce a selective marker gene to each of the two or more alleles:
(i) a genome engineering system comprising a sequence-specific nucleic acid cleaving molecule capable of targeting a target region in the two or more alleles in the chromosomal genome and cleaving the target region, or a polynucleotide encoding the sequence-specific nucleic acid cleaving molecule, and
(ii) two or more donor DNAs for selective markers, each of which has an upstream homology arm having a nucleotide sequence that can be homologously recombined with an upstream nucleotide sequence of the target region and a downstream homology arm having a nucleotide sequence that can be homologously recombined with a downstream nucleotide sequence of the target region, and comprises a nucleotide sequence of the selective marker gene between the upstream homology arm and the downstream homology arm, the two or more donor DNAs for selective markers respectively having distinguishably different selective marker genes, wherein the selective marker gene is unique to each type of donor DNA for the selective marker, and the number of types of the donor DNAs for selective markers is equal to or more than the number of the alleles that are subject to genome engineering; and (b) after the step (a), selecting a cell having the distinguishably different unique selective marker genes, which are respectively introduced in the two or more alleles by respective homologous recombination of the two or more alleles with different types of donor DNAs for selective markers, and expressing all the distinguishably different selective marker genes thus introduced (step for positive selection).

2. A method for engineering two or more alleles in the chromosomal genome, comprising the steps of:
(a) introducing the following (i) and (ii) to a cell comprising two or more alleles to introduce a selective marker gene to each of the two or more alleles:
(i) a genome engineering system comprising a sequence-specific nucleic acid cleaving molecule capable of targeting a target region in the two or more alleles in the chromosomal genome and cleaving the target region, or a polynucleotide encoding the sequence-specific nucleic acid cleaving molecule, and
(ii) two or more donor DNAs for selective markers, each of which has an upstream homology arm having a nucleotide sequence that can be homologously recombined with an upstream nucleotide sequence of the target region and a downstream homology arm having a nucleotide sequence that can be homologously recombined with a downstream nucleotide sequence of the target region, and comprises a nucleotide sequence of the selective marker gene between the upstream homology arm and the downstream homology arm, the two or more donor DNAs for selective markers respectively having distinguishably different selective marker genes, wherein the selective marker gene is unique to each type of donor DNA for the selective marker, and the number of types of the donor DNAs for selective markers is equal to or more than the number of the alleles that are subject to genome engineering; and (b) after the step (a), selecting a cell having the distinguishably different unique selective marker genes, which are respectively introduced in the two or more alleles by respective homologous recombination of the two or more alleles with different types of donor DNAs for selective markers, and expressing all the distinguishably different selective marker genes thus introduced (step for positive selection).

3. The method according to claim 1 or 2, wherein the target region has a length of 5 kbp or more.

4. The method according to claim 3, wherein the target region has a length of 8 kbp or more.

5. The method according to any one of claims 1 to 4, wherein each of the two or more donor DNAs for selective markers has a selective marker gene for positive selection, a marker gene for negative selection, and a target sequence between the upstream homology arm and the downstream homology arm, wherein in the case of using the selective marker gene both for positive selection and for negative selection, another selective marker gene for negative selection is optionally absent,
the method further comprising the steps of:
(c) after the step (b), introducing the following (iii) and (iv) to the selected cell to introduce a donor DNA for recombination to each of the two or more alleles:
(iii) a genome engineering system comprising a sequence-specific nucleic acid cleaving molecule capable of targeting the target sequence and cleaving the target sequence, or a polynucleotide encoding the sequence-specific nucleic acid cleaving molecule, and
(iv) a donor DNA for recombination comprising a desired nucleotide sequence, the donor DNA for recombination having an upstream homology arm having a nucleotide sequence that can be homologously recombined with an upstream nucleotide sequence of the target region and a downstream homology arm having a nucleotide sequence that can be homologously recombined with a downstream nucleotide sequence of the target region; and (d) after the step (c), selecting a cell not expressing the marker gene for negative selection (step for negative selection).

6. The method according to claim 3, wherein
each of the two or more donor DNAs for selective markers has a selective marker gene for positive selection, a marker gene for negative selection, and a target sequence between the upstream homology arm and the downstream homology arm, wherein in the case of using the selective marker gene both for positive selection and for negative selection, another selective marker gene for negative selection is optionally absent,
the method further comprising the steps of:
(c) after the step (b), introducing the following (iii) and (iv) to the selected cell to introduce a donor DNA for recombination to each of the two or more alleles:
(iii) a genome engineering system comprising a sequence-specific nucleic acid cleaving molecule capable of targeting the target sequence and cleaving the target sequence, or a polynucleotide encoding the sequence-specific nucleic acid cleaving molecule, and
(iv) a donor DNA for recombination comprising a desired nucleotide sequence, the donor DNA for recombination having an upstream homology arm having a nucleotide sequence that can be homologously recombined with an upstream nucleotide sequence of the target region and a downstream homology arm having a nucleotide sequence that can be homologously recombined with a downstream nucleotide sequence of the target region; and (d) after the step (c), selecting a cell not expressing the marker gene for negative selection (step for negative selection).

7. The method according to claim 4, wherein
each of the two or more donor DNAs for selective markers has a selective marker gene for positive selection, a marker gene for negative selection, and a target sequence between the upstream homology arm and the downstream homology arm, wherein in the case of using the selective marker gene both for positive selection and for negative selection, another selective marker gene for negative selection is optionally absent,
the method further comprising the steps of:
(c) after the step (b), introducing the following (iii) and (iv) to the selected cell to introduce a donor DNA for recombination to each of the two or more alleles:
(iii) a genome engineering system comprising a sequence-specific nucleic acid cleaving molecule capable of targeting the target sequence and cleaving the target sequence, or a polynucleotide encoding the sequence-specific nucleic acid cleaving molecule, and
(iv) a donor DNA for recombination comprising a desired nucleotide sequence, the donor DNA for recombination having an upstream homology arm having a nucleotide sequence that can be homologously recombined with an upstream nucleotide sequence of the target region and a downstream homology arm having a nucleotide sequence that can be homologously recombined with a downstream nucleotide sequence of the target region; and (d) after the step (c), selecting a cell not expressing the marker gene for negative selection (step for negative selection).

8. The method according to any one of claims 5 to 7, wherein the region between the upstream homology arm and the downstream homology arm of the donor DNA for recombination has a length of 5 kbp or more.

9. The method according to claim 6 or 7, wherein the region between the upstream homology arm and the downstream homology arm of the donor DNA for recombination has a length of 5 kbp or more.

10. The method according to claim 7, wherein the region between the upstream homology arm and the downstream homology arm of the donor DNA for recombination has a length of 5 kbp or more.

11. The method according to claim 8, wherein the region between the upstream homology arm and the downstream homology arm of the donor DNA for recombination has a length of 8 kbp or more.

12. A genome engineering kit for engineering two or more alleles in the chromosomal genome, comprising the following (i) and (ii):
(i) a genome engineering system comprising a sequence-specific nucleic acid cleaving molecule capable of targeting a target region in the chromosomal genome and cleaving the target region, or a polynucleotide encoding the sequence-specific nucleic acid cleaving molecule, and
(ii) two or more donor DNAs for selective markers, each of which has an upstream homology arm having a nucleotide sequence that can be homologously recombined with an upstream nucleotide sequence of the target region and a downstream homology arm having a nucleotide sequence that can be homologously recombined with a downstream nucleotide sequence of the target region, and comprises a nucleotide sequence of a selective marker gene between the upstream homology arm and the downstream homology arm, the two or more donor DNAs for selective markers respectively having distinguishably different selective marker genes, wherein the selective marker gene is unique to each type of donor DNA for the selective marker, and the number of types of the donor DNAs for selective markers is equal to or more than the number of the alleles that are subject to genome engineering.

13. The kit according to claim 12, wherein the target region has a length of 5 kbp or more.

14. The kit according to claim 13, wherein the target region has a length of 8 kbp or more.

15. The kit according to any one of claims 12 to 14, further comprising a donor DNA for recombination.

16. The kit according to any one of claims 12 to 15, wherein a region between an upstream homology arm and a downstream homology arm of the donor DNA for recombination has a length of 5 kbp or more.

17. The kit according to any one of claims 12 to 16, wherein a region between an upstream homology arm and a downstream homology arm of the donor DNA for recombination has a length of 8 kbp or more.

18. The kit according to claim 12, wherein the target region has a length of 5 kbp or more, and a region between an upstream homology arm and a downstream homology arm of the donor DNA for recombination has a length of 5 kbp or more.

19. The kit according to claim 18, wherein the target region has a length of 8 kbp or more, and a region between an upstream homology arm and a downstream homology arm of the donor DNA for recombination has a length of 8 kbp or more.

20. The method according to claim 5, wherein the donor DNA for recombination has no nucleotide sequence in the region between the upstream homology arm and the downstream homology arm of the donor DNA for recombination, and the upstream and downstream sequences of the target region are seamlessly linked, without insertion, substitution and deletion of a base, in the thus-engineered two or more alleles in the chromosomal genome.

21. The method according to claim 6 or 7, wherein the donor DNA for recombination has no nucleotide sequence in the region between the upstream homology arm and the downstream homology arm of the donor DNA for recombination, and the upstream and downstream sequences of the target region are seamlessly linked, without insertion, substitution and deletion of a base, in the thus-engineered two or more alleles in the chromosomal genome.

22. The method according to claim 3, wherein a target sequence of site-specific recombinase is absent in the thus-engineered two or more alleles in the chromosomal genome.

23. The method according to claim 4, wherein a target sequence of site-specific recombinase is absent in the thus-engineered two or more alleles in the chromosomal genome.

24. The method according to claim 5, wherein a target sequence of site-specific recombinase is absent in the thus-engineered two or more alleles in the chromosomal genome.

25. The method according to claim 6 or 7, wherein a target sequence of site-specific recombinase is absent in the thus-engineered two or more alleles in the chromosomal genome.

26. The method according to any one of claims 1 to 11 and 20 to 25, wherein in the step (b), single-cell cloning is not performed in a process up to the selection of the cell in which the two or more alleles are engineered.

27. A cell having two or more alleles in the chromosomal genome in relation to a target region, wherein the respective target regions of the two or more alleles are deleted, and the upstream and downstream sequences of the target region are seamlessly linked without insertion, substitution and deletion of a base.

28. The cell according to claim 27, wherein the target region has a length of 5 kbp or more.

29. The cell according to claim 27 or 28, wherein the cell has no target sequence of site-specific recombinase in the genome.

30. The method according to claim 6 or 7, wherein the region between the upstream homology arm and the downstream homology arm of the donor DNA for recombination has a length of 8 kbp or more.

31. The kit according to claim 15, wherein the donor DNA for recombination has no nucleotide sequence in the region between the upstream homology arm and the downstream homology arm of the donor DNA for recombination.
